# EUROPÄISCHE PATENTSCHRIFT

(11) **EP 3 937 777 B1**
(45) Veröffentlichungstag und Bekanntmachung des Hinweises auf die Patenterteilung: **20.11.2024**
(21) Anmeldenummer: 20712485.0
(22) Anmeldetag: 12.03.2020
(51) Int. Cl.: A61B 5/11, A61B 5/00, A61C 19/045

(54) **VORRICHTUNG ZUR BEWEGUNGSVERFOLGUNG DES KIEFERS**
DEVICE FOR TRACKING MOVEMENT OF THE JAW
DISPOSITIF DE SUIVI DE MOUVEMENTS DE MÂCHOIRE

(30) Priorität: 12.03.2019 DE 102019106310
(43) Veröffentlichungstag der Anmeldung: 19.01.2022
(73) Patentinhaber: Forstgarten International Holding GmbH, 9016 St. Gallen (CH)
(72) Erfinder: KALTENBACH, Stefan, 9016 St. Gallen (CH); LANDGRAF, Johannes, 9016 St. Gallen (CH)
(74) Vertreter: Schmidt, Christian
(86) Internationale Anmeldenummer: PCT/EP2020/056716
(87) Internationale Veröffentlichungsnummer: WO 2020/182962

(56) Entgegenhaltungen:
- WO-A1-2016/044251
- JP-B2- 4 665 051
- US-A1- 2016 331 316
- "IEEE Recommended Practice for Inertial Sensor Test Equipment, Instrumentation, Data Acquisition, and Analysis;IEEE Std 1554-2005 ED - Anonymous", IEEE STANDARD; [IEEE STANDARD], IEEE, PISCATAWAY, NJ, USA, 1 January 2005 (2005-01-01), pages _1 - 103, XP017603831, ISBN: 978-0-7381-4738-3

## Beschreibung

### Gebiet der Erfindung

Die Erfindung betrifft die messtechnische Erfassung von komplexen Kieferpositionen und von Bewegungen des Kiefers und des Kopfes durch intraorale Vorrichtung oder durch am Gebiss montierte Vorrichtungen kurzzeitig oder länger andauernd, insbesondere für mehrere Stunden dauernde Bewegungsanalysen bei Belastung, bei Stress und beim Schlafen.

### Hintergrund

Die folgenden Ausführungen sind als Hintergrund zu der offenbarten Erfindung zu verstehen und nicht als Eingeständnis, dass irgendeines der diskutierten Merkmale Stand der Technik darstellt. Des Weiteren können in diesem Abschnitt offenbarte Merkmale mit weiteren Merkmale auch aus anderen Abschnitten der vorliegenden Offenbarung kombiniert werden.

Die Dimensionen bzw. Freiheitsgrade der Bewegung umfassen allgemein drei aufeinander orthogonal stehende Translationsrichtungen und drei aufeinander senkrecht stehende Rotationsachsen. In diesem Zusammenhang benutzt man die physikalische Bezeichnung 6-Degrees-of-Freedom, um drei translatorische Positionskoordinaten und drei rotatorische Raumorientierungskoordinaten zu definieren. Bewegungen von realen Körpern sind durch die Veränderung der 6-Degrees-of-Freedom Koordinaten gekennzeichnet.

Bewegung kann man durch Ankopplung von Messvorrichtungen an bewegte Objekte messen. Misst man die Bewegung des Oberkiefers, so hat man damit die Bewegung des Kopfes, soweit sich dieser nicht deformiert und umgekehrt. Misst man die Bewegung des unteren Zahnbogens oder auch nur eines Implantates im Unterkiefer, so hat man die Bewegung des Unterkiefers erfasst, solange sich das erfasste Teilobjekt (zum Beispiel Implantat) mit dem größeren Objekt (zum Beispiel Unterkiefer) ohne Relativbewegung mitbewegt. Die fixierte Ankopplung ist also eine mitentscheidende Voraussetzung für eine hohe Messgenauigkeit. Die Lage und Orientierung des Körperteils ergeben sich aus der Lage und Orientierung des in ihrer Bewegung erfassten Vorrichtung durch 6-Degrees-of-Freedom Koordinatentransformation.

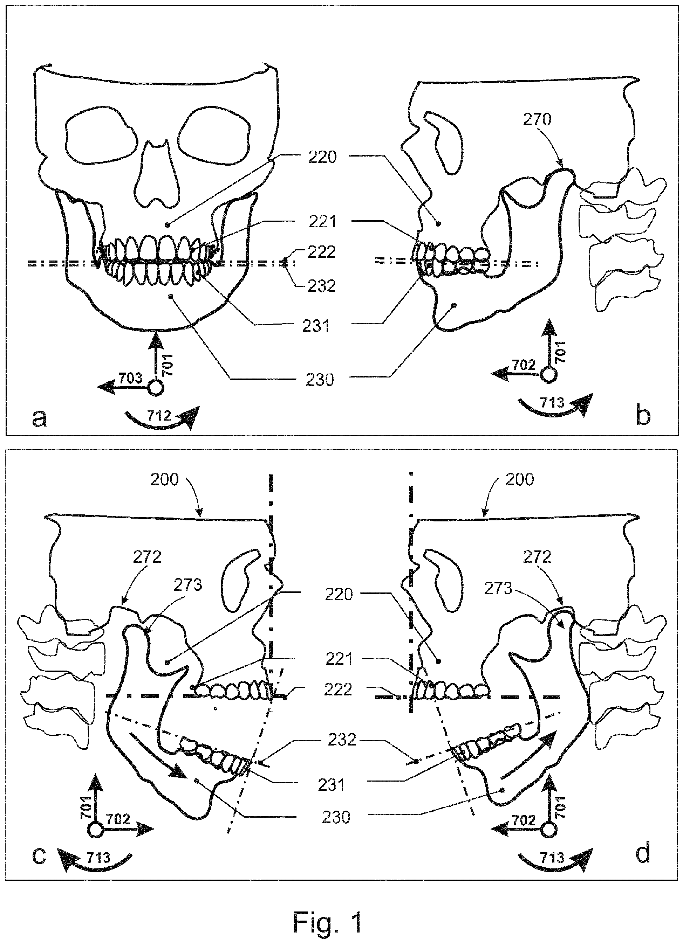

Insbesondere in der Biomechanik sind Relativpositionen und Relativbewegungen zwischen Körperteilen zu erfassen. Im Folgenden wird dies am Beispiel des Kiefers und der Zahnbögen beschrieben. Hier wird z.B. sowohl die Position bzw. Bewegung des Oberkiefers OK erfasst als auch die Position bzw. Bewegung des Unterkiefers UK. Die Relativbewegung des Unterkiefers UK relativ zum Oberkiefer OK ergibt sich durch Koordinaten-Transformation in das bewegte Bezugssystem des Oberkiefers. Das Bezugssystem des Oberkiefers OK mit seinen drei orthogonalen Raumachsen ist in Fig. 1, sowie Fig. 3 bis Fig. 11 schematisch eingezeichnet. In der Sagittalebene des Gebisses betrachtet zeigt das Bezugssystem zwei Raumrichtungen, nämlich 701 nach oben und 702 nach vorne. Die Öffnungsbewegung des Unterkiefers folgt vorwiegend der Drehrichtung 713, wobei es sich hier genau genommen um komplexe sechsdimensionale Bewegung handelt, die aus, zum Beispiel mehreren Translationen und Rotationen überlagert ist. Dem Fachmann ist vertraut, sechsdimensionale Bewegungen als sechsdimensionale Matrizen darzustellen und Relativbewegungen ebenfalls sechsdimensional in Matrizenform zu beschreiben.

Im Zusammenhang mit den Bewegungen des Kauapparates, des Kiefergelenks und/oder der Zähne zueinander sind viele unterschiedliche Vorrichtungen und Verfahren vorgeschlagen worden.

Erwähnenswert sind z.B. die Vorrichtungen der Firma Zebris, die die Bewegung des Unterkiefers über ein Kopplungselement auf eine Unterkiefergabel überträgt, wobei die Bewegung dieser Unterkiefergabel durch ein Messystem erfasst wird, das am Kopf oberhalb der Nase befestigt ist. Die Erfassung der Relativbewegung erfolgt also direkt durch die z.B. optischen oberen Sensoren in deren Position relativ zu den Markern an der Unterkiefergabel.

Ebenfalls von Bedeutung im Markt sind die Systeme der Firmen DDI und Orangedental zur Bewegungsmessung mit Digitalkameras, wobei sowohl die Bewegung des Unterkiefers als auch die des Oberkiefers bzw. des Kopfes von einem Kamerasystem gemessen werden, das fest im Raum montiert ist. Hier misst man auch die Kopfbewegung, getrennt von der Bewegung des Unterkiefers, die Relativbewegung ergibt sich aus der Koordinatentransformation der Positionen des Unterkiefers in das ggfs. bewegte Koordinatensystem des Oberkiefers bzw. Kopfes.

Eine Mischform wird von der Firma Planmeca angeboten. Eine Brille trägt mehrere Markerkugeln und ein vom Unterkiefer nach außen geführter kleiner Messbogen trägt auch mehrere Markerkugeln, jeweils als Markersysteme, die sich mit der Kaubewegung und der Mundöffnung relativ zueinander bewegen, Die Erfassung erfolgt mit mehreren Kameras schräg von vorne. Die Kameras sind als Besonderheit in ein DVT Kopfröntgengerät integriert. Die Markersysteme sind so eng anliegend, dass sie die Kreisfahrt des DVT Kamerabogens nicht behindern.

Darüber hinaus sind Messverfahren im Handel verfügbar, mit denen ein Tracking insbesondere für Protrusionsschienen möglich ist, die gegen Schnarchen und Schlafapnoe verordnet werden. Dabei ist in einem Teil der Schiene ein Schwerkraftsensor integriert, der erfasst, in welcher Raumlage sich die Schiene befindet und ob zufällige natürliche Kopf und Kieferbewegungen vorhanden sind. Diese Vorrichtungen sind jedoch zur Erfassung der relativen Bewegungs- und Positionsdaten zwischen Oberkiefer und Unterkiefer nicht sehr geeignet, weil das Problem der sich aufaddierenden Summe der Messfehler z.B. bei wiederholt zum Ausgangspunkt zurück kehrenden Translations- und Rotationsbewegungen derzeit nicht gelöst ist.

Für andere Körperteile, die in der Bewegung erfasst werden, z.B. bei den Beinen, kann man Strümpfe mit Markierungen anbringen, um diese Markierungen beim Sport zu verfolgen. Dies geht bei der Mundschleimhaut nicht und die Verfolgung markierter Zähne mit Kameras von außen ist nicht möglich, wenn der Mund geschlossen ist. Würde man die Schneidezähne markieren und die Lippen abheben, würde nach kurzer Zeit ein sehr unnatürliches Verhalten entstehen, also ganz anders als beim Sportler mit den Markerstrümpfen an den Beinen.

Die präzisen Messmethoden, die beschrieben, teilweise zum Patent angemeldet und teilweise auch im Markt sind, arbeiten durchweg mit nach außen geführten Gestellen, Gabeln oder Bügeln als Teil des Markersystems. Diese nach außen geführten Vorrichtungen sind grundsätzlich nicht geeignet, beim Schlafen oder bei der Arbeit, insbesondere zur Bewegungserfassung in Stresssituationen etc. Anwendung zu finden, weil sie dabei deformiert oder sogar zerstört werden würden und zudem die natürliche Bewegung des Körpers behindern oder Bewegungsabläufe verändern.

Alternative Messverfahren wie z.B. Ignident, benutzen einen Magnetfeldgenerator, der oberhalb des Kopfes angebracht ist und der nutzbares und/oder bekanntes Magnetfeld im Mundraum erzeugt. Folglich können im Mund befindliche Magnetfeldsensoren, meist ein Sensor für den Unterkiefer und/oder ein Sensor für den Oberkiefer, die lokalen Magnetfeldstärken erfassen und daraus die 6-Degrees-of-Freedom Koordinaten relativ zum Magnetfeldgenerator bestimmen. In der Praxis bedeutet dies, dass der Patient oder Proband sich während der gesamten Messdauer in dem räumlich sehr eng begrenzten auswertbaren Magnetfeldbereich unterhalb des Magnetfeldgenerators befinden muss, was auch nicht über einen längeren Zeitraum praktikabel ist.

Die Patentanmeldung von Forstgarten schließlich (WO 2016/142264 A1) offenbart eine innen liegende Markierung oder einen Sensor, die bzw. der am Unterkiefer befestigt ist und die bzw. der, z.B. mit einer Kamera vom Oberkiefer, aus erfassbar wäre, allerdings stört hier der Volumenverbrauch und die optisch störende Zunge in der Praxis. Zudem ist die Frage der Energieversorgung für einen autark intraoral arbeitenden Sensor - ohne Leistungszufuhr von außen - nicht gelöst.

Das vorherrschende Problem der Integralsensoren, die für solche Anwendungen verwendet werden könnten, liegt darin, dass sie entweder eine zu grobe Auflösung und einen zu großen Messbereich haben, so dass sie die feinen Kontaktbewegungen und Kontaktpositionen - etwa die von Oberkiefer und/oder Unterkiefer mit kleinen Geschwindigkeiten, kleinen Beschleunigungen und/oder kleiner Amplitude - nicht korrekt erfassen können. Oder sie haben eine so kleine Auflösung und oder einen so kleinen Messbereich, so dass sie die ausladenden Relativbewegungen - etwa die von Oberkiefer und/oder Unterkiefer mit großen Geschwindigkeiten, großen Beschleunigungen und/oder großer Amplitude - beispielsweise beim Gähnen nicht korrekt erfassen können. In der Praxis benötigt man aber beide Messbereiche mit voller oder großer Genauigkeit. Integralsensoren sind Sensoren, bei denen das erzeugte Signal charakteristisch für eine Kenngröße (z.B. die Beschleunigung) ist, wobei für die Bestimmung der oder einer zu bestimmenden Messgröße (z.B. der Position und/oder der Geschwindigkeit) zumindest eine Integration, insbesondere über die Zeit, erforderlich ist oder ggf. mehrere Integrationsprozesse durchgeführt werden müssen.

Bei Integralsensoren können besondere Probleme auftreten, weil bei der Integration mit verfügbaren Sensoren, die die Beschleunigung und/oder die Geschwindigkeiten oder Drehraten messen, entweder die besonders langsamen Bewegungen im Integral nicht korrekt erfasst werden und oder auch die besonders schnellen Bewegungen nicht korrekt erfasst werden. Im Resultat ist das Integral der erfassten Bewegung bei einer komplexen Bewegung wie der des Unterkiefers relativ zum Oberkiefer nicht exakt korrekt. In der Folge ist das Integral einer wiederholt zurückkehrenden Bewegung wie der Kaubewegung oder der Bewegung des Kopfes und des Kiefers beim Schlafen nicht korrekt und führt zu fortlaufend ansteigenden Fehlkalkulationen bezüglich der Position von Oberkiefer und Unterkiefer bzw. deren Relativposition.

Eine weitere Vorrichtung zur Erfassung von Kieferbewegungen ist aus der JP4665051B bekannt.

Damit fehlt bislang jede Möglichkeit, eine wirklich präzise Messung der Kieferbewegung - Unterkiefer und Oberkiefer relativ zueinander - und gleichzeitig der Kopfbewegung im Raum, zum Beispiel im Sinne einer zeitlich lange fortgesetzten 6-Degrees-of-Freedom Erfassung, in natürlicher ungestörter Situation, z.B. beim Schlafen durchzuführen, vorzugsweise ohne die natürlichen Bewegungen wesentlich zu verändern oder zu behindern.

### Aufgabe der Erfindung

Eine grundsätzliche Aufgabe der Erfindung ist es, Verbesserungen im Bereich der Bewegungsverfolgung von Körperteilen anzugeben.

Eine weitere oder alternative zu lösende Aufgabe besteht darin, eine praxisgerechte Vorrichtung, ein System und ein Verfahren bereitzustellen, das eine Erfassung von Positionen und Bewegungen des Unterkiefers und des Kopfes und/oder des Oberkiefers in kurzen Messzeiträumen, etwa einige Sekunden oder Minuten, aber auch in langen Messzeiträumen von mehreren Stunden unter möglichst natürlichen Bedingungen erlaubt oder erleichtert. Das System soll wegen der möglichst natürlichen Umstände im Mund integriert oder integrierbar sein. Die Messung soll möglich sein, ohne dass außen um den Kopf herum aufwändige Empfänger oder Sender installiert werden müssen. Die Erfindung ist allerdings nicht notwendigerweise darauf beschränkt. Da vorwiegend starre Befestigungsvorrichtungen die Bewegung in ihren sechsdimensionalen Koordinaten auf andere Positionen übertragen, können die im Rahmen der Erfindung einsetzbaren Bewegungssensoren auch an Befestigungsvorrichtungen angebracht sein, die dann ihrerseits an den Körperteilen angebracht sind bzw. zur entsprechenden Anbringung vorgesehen sind. Zum Beispiel können die Befestigungsvorrichtungen am Oberkiefer bzw. am Unterkiefer angebracht sein. Darüber hinaus sollen für die langandauernde Anwendung keine empfindlichen und störenden Messbügel aus dem Mund nach außen geführt oder außen aufgesetzt werden.

Eine weitere oder alternative zu lösende Aufgabe liegt darin, endlich eine genaue Erfassung der feinen Kontaktbewegungen der Zähne, beispielsweise beim Kauen, zu erleichtern oder zu ermöglichen und/oder eine genaue Erfassung der weit ausholenden Bewegung der Kiefer, beispielsweise beim Gähnen oder beim Schreien, zu erleichtern oder zu ermöglichen, vorzugsweise mit dem gleichen System und/oder der gleichen Sensorik, vorzugsweise integrierenden Sensoren. Dies ist nicht trivial, weil dies mit bisher verfügbaren integrierenden Sensoren nicht beides gleichzeitig gelingt, also Messung der feinen und der ausholenden Bewegung. Die Problematik zeigt sich besonders darin, dass entweder die schnellen und oder die langsamen Bewegungen oder die Bewegungen mit Extremwerten der Geschwindigkeit und/oder Beschleunigung nicht korrekt erfasst werden und folglich die messtechnisch erfasste Bahnkurve der zusammengesetzten translatorischen und rotatorischen Bewegung nicht exakt der realen Bewegung entspricht. In der direkten Folge führt eine real an eine definierbaren Punkt führende Bewegung in der Messung zu einem um eine messbare Translations- und oder Rotationsdifferenz verschiedenen scheinbaren Endpunkt. Solche Fehler addieren sich im Laufe längerer Bewegungsverfolgung auf. Nun besteht eine zu lösende Aufgabe darin, ein System und ein Verfahren anzubieten, mit dessen Hilfe das Problem der sich aufsummierenden Messfehler gelöst werden kann.

Ein Anwendungsziel kann insbesondere darin bestehen, die 6-Degrees-of-Freedom Bewegungserfassung im Kiefergelenk und am Kopf beim Sport, bei stressig belastender Arbeit und/oder im Bett liegend, wie z.B. beim Schlafen, durchführen zu können. Besonders bevorzugt ist eine Lösung, mit deren Hilfe ein Patient zuhause oder in der Klinik tags und nachts in seinen Kieferbewegungen und Kopfbewegungen verfolgt werden kann, ohne dass störendes Gerät am Kopf angebracht ist. Dabei ist es von Vorteil, insbesondere als Nebenziel, dass die Aktivität der Sensorik auch hinsichtlich Energieverbrauch so weit sichergestellt ist, dass die Messung über mindestens eine oder einige Stunden durchführbar ist, wobei eine Energiezufuhr von außen nicht durch bewegungsbehindernde Kabel erfolgen sollte.

### Vorgeschlagene Lösung(en)

Mindestens eine der oben genannten Aufgaben wird durch den Gegenstand des unabhängigen Anspruchs gelöst. Vorteilhafte Weiterbildungen sind in den Unteransprüchen gekennzeichnet. Der Wortlaut sämtlicher Ansprüche wird hiermit durch Bezugnahme zum Inhalt dieser Beschreibung gemacht. Die Verwendung der Einzahl soll die Mehrzahl nicht ausschließen, was auch im umgekehrten Sinn zu gelten hat, soweit nichts Gegenteiliges offenbart ist. Außerdem sei angemerkt, dass die Offenbarung nicht durch die Ansprüche beschränkt ist sondern andere, derzeit nicht beanspruchte vorteilhafte Konzepte und Lösungen beinhalten kann.

Im Folgenden werden einzelne Vorrichtungen und Verfahrensschritte und das System näher beschrieben. Bei der Beschreibung der Vorrichtung kann der Bezug der Körperteile, die sich relativ zueinander bewegen, z.B. Oberkiefer OK und Unterkiefer UK vertauscht werden. Denn wenn der Unterkiefer sich relativ zum Oberkiefer bewegt, so ist das gleichbedeutend mit einer Relativbewegung des Oberkiefers zum Unterkiefer. Alle Körperteile wiederum können sich mit sechs Freiheitsgraden im Realraum bewegen. Zudem kann anstelle der Bewegung der Zahnbögen wahlweise die Bewegung des Oberkiefers erfasst werden oder die Bewegung des Schädelknochens, da dieser im Normalfall knöchern fest mit dem Oberkiefer verbunden ist.

Einsatzgebiete der vorgeschlagenen Lösung sind insbesondere Diagnostik, Analyse, Training und Therapie im Zusammenhang mit Kiefergelenksfunktionen und Gebissfunktionen, insbesondere mit der statischen und dynamischen Position und Bewegung des Unterkiefers relativ zum Oberkiefer und den damit verbundenen Kiefergelenkszuständen. Besondere Anwendung haben diese Messungen für die digitale Planung und Fertigung von Zahnersatz, von kieferorthopädischen Vorrichtungen und für den Einsatz in der Schlafmedizin, z.B. bei Schlafapnoe, die dadurch gekennzeichnet ist, dass Unterkiefer und Zunge im Schlaf entspannt nach hinten fallen und dabei die Atemwege verschließen.

Eine mit der anspruchsgemäßen Lösung erreichbare Besonderheit liegt darin, dass die Wirkungsweise mindestens zweier unterschiedlicher Sensoren oder zweier Sensortechnologien in einem System so kombiniert werden kann, dass während der Bewegung oder nach Bewegungsabschnitten eine Korrektur der sich ansonsten aufaddierenden Messfehler der Bewegungserfassung erfolgen kann. Insbesondere kann das System für eine Bahnkorrektur und/oder eine Korrektur der Messwerte für Geschwindigkeit und/oder Beschleunigung sowohl für Translation als auch für Rotation und mit Vorzug zusammengesetzte Bewegungen in bis zu sechs Freiheitsgraden ausgebildet sein. Das vorgeschlagene System zur Bewegungsmessung und/oder -verfolgung umfasst mindestens ein erstes Sensorsystem S_motion (Bewegungssensorsystem), beispielsweise zur Erfassung von Beschleunigung und/oder Geschwindigkeit. Alternativ oder ergänzend kann direkt oder indirekt auch der Weg erfasst werden, wobei dieses die ganze Bahnkurve erfassen kann oder einen größeren Bereich der Bahnkurve eines Körperteils im Raum oder relativ zu einem weiteren Körperteil. Bevorzugt umfasst das vorgeschlagene System noch mindestens ein zweites Sensorsystem (z.B. S_calibration oder Kalibriersensorsystem). Dieses zweite Sensorsystem erfasst einen Teilbereich, vorzugsweise nur einen Teilbereich, des von S_motion erfassten Bewegungsbereiches. Das zweite Sensorsystem hat in dem Teilbereich mit Vorzug einen Bereich hoher Messgenauigkeit in der Erfassung mindestens der Position und/oder Raumorientierung des Körpers. Der Bereich hoher Messgenauigkeit kann durch die Konstruktion des Sensorsystems intrinsisch vorgegeben oder durch den Hersteller festgelegt sein. Die Genauigkeit des zweiten Sensorsystems ist bevorzugt höher als die mit dem ersten Sensorsystem erzielbare Genauigkeit. Bevorzugt arbeitet S_calibration mit mindestens zweifacher Genauigkeit des ersten Sensorsystems Scalibration, besonders bevorzugt mit mindestens vierfacher Genauigkeit. Die relativ zueinander beweglichen Körperteile tragen bevorzugt Teile des ersten Sensorsystems S_motion und auch Teile des zweiten Sensorsystems S_calibration. Sobald sich der bewegte Körper in dem Bereich der hohen Messgenauigkeit des zweiten Sensorsystems S_calibration befindet, wird mindestens ein Messergebnis von S_calibration dazu verwendet, eine Rekalibrierung oder eine Korrektur der Messwerte des Sensorsystems S_motion zu veranlassen oder durchzuführen. Die durchgeführte Korrektur erreicht, dass die Messwerte oder die anhand der Messwerte des ersten Sensorsystem S_motion ermittelten Werte für z.B. Beschleunigung, Geschwindigkeit und/oder translatorische und rotatorische Bahnkurve, an die genaueren Messwerte des Sensorsystems S_calibration angepasst werden. Dadurch wird eine etwaige Abweichung der mittels S_motion ermittelten Bewegungskoordinaten von der realen Bewegung ganz oder teilweise korrigiert. Bevorzugt ist das System so ausgebildet, dass zudem eine Rekalibrierung der Sensorparameter anhand der Informationen des Sensorsystems S_calibration erfolgen kann. Besonders bevorzugt erfolgt mittels einer Prozessoreinheit oder -einrichtung und einer Speichervorrichtung eine insbesondere auch zeitlich rückwirkende Korrektur der erhaltenen Messwerte des ersten Sensorsystems S_motion, zum Beispiel in dem Sinne dass sich zumindest in der nullten Ableitung des Ortes eine Angleichung der Bahnkurve an die vom zweiten Sensorsystem S_calibration ermittelte mindestens eine Position ergibt. Bevorzugt erfolgt auch die Angleichung in der Geschwindigkeit und/oder in der Beschleunigung. Dadurch werden sprunghaft erscheinende Auswirkungen der Korrektur ausgeglichen und es wird eine der Realität möglichst nahe entsprechende translatorische und rotatorische Bahnkurve ermittelt. Würde man diese angleichende Korrektur nicht vornehmen, so bestünde die Gefahr dass die mittels S_calibration korrigierte Abfolge der sechsdimensionalen Bahnkurve sprunghaft erscheint. Das könnte als körperbaubedingte oder krankheitsbedingte Bewegungsstörung mit sprunghafter Beschleunigung falsch interpretiert werden. Durch die vorgeschlagene Korrektur wird die eine hohe Genauigkeit in der Position erreicht. In der erweiterten Angleichung kann die möglichst realgetreue Erfassung der Geschwindigkeit und der Beschleunigung vereinfacht werden.

Durch diese während der Messzeit oder Betriebszeit mit Vorzug wiederholt - zweckmäßigerweise auseichend oft für eine gewünschte Genauigkeit - erfolgende Korrektur/Kalibrierung kann die Bewegung bzw. die Relativbewegung über einen großen Zeitraum, z.B. 8 Stunden oder mehr, mit hoher Genauigkeit erfasst werden. Für den Dentalbereich typisch sind z.B. Anforderungen, die Bewegung auf zehn bis hundert Mikrometer genau zu erfassen, im Allgemeinen zwischen ein und tausend Mikrometer genau. Eine derart hohe Präzision ist vorteilhaft oder sogar erforderlich, weil Positionsänderungen von zehn bis hundert Mikrometer für die Kontaktausbildung an den Zähnen maßgeblich sind. Für die Bewegungserfassung im Schlaf sind des weiteren Messzeiten von bis zu zwölf Stunden typisch, was letztlich dazu führt, dass die geforderte Genauigkeit auch nach zwölf Stunden noch vorhanden sein soll. Während dies mit bisher verfügbaren Integralsensoren absolut unmöglich ist, ist die vorgeschlagene Lösung in der Lage, diese Anforderungen zu erfüllen. Mit zur vorgeschlagenen Lösung gehört bevorzugt eine Vorrichtung oder ein System zur Energieversorgung des zumindest teilweise aktiven Sensorsystems. In der bevorzugten Ausführung werden die Erfassungsdaten entweder zwischengespeichert, zum Beispiel in der Vorrichtung etwa einem Datenspeicher, und nach der Messphase an ein Auswertesystem übertragen oder die Übertragung erfolgt während der Messung bevorzugt kabellos, bevorzugt über einen der verfügbaren Funkstandards.

Vorteilhafte Weiterbildungen und Ausgestaltungen sind Gegenstand der abhängigen Ansprüche und/oder ergeben sich aus der folgenden Beschreibung in Verbindung mit den Figuren.

### Liste der Figuren

Fig. 1 zeigt den Schädel 200 mit Oberkiefer 220 und Unterkiefer 230 und Zahnbögen 221, 231 und schematisch eingezeichnet das im Grunde immer sechsdimensionale Koordinatensystem für Translationen und Rotationen

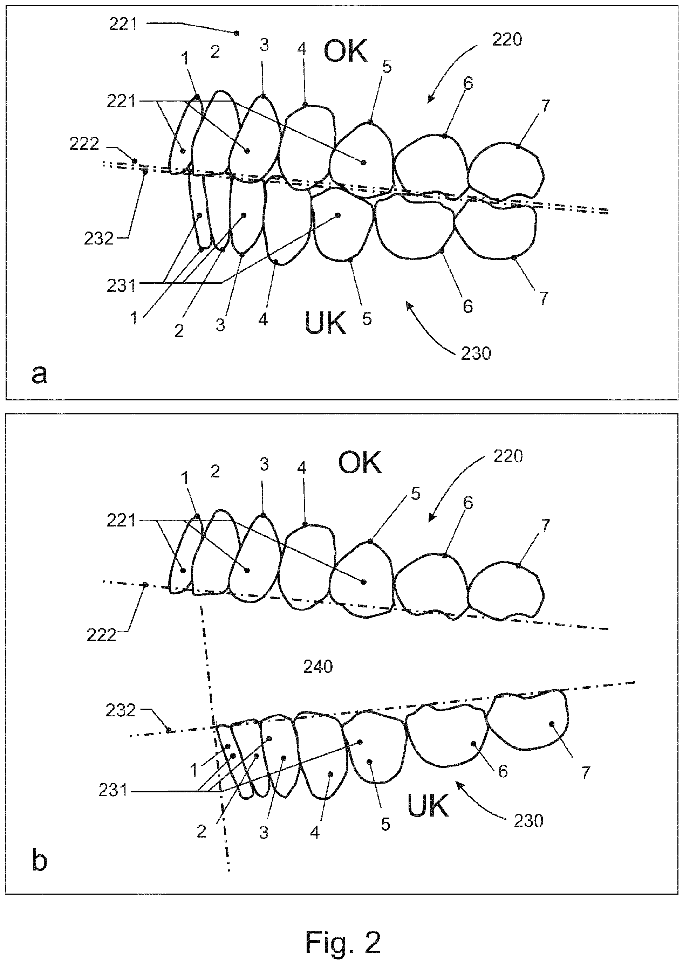

Fig. 2 zeigt die Zahnbögen 221, 231 des Oberkiefers 220 und des Unterkiefers 230, oben Fig. 2a geschlossen und unten in Fig. 2b geöffnet.

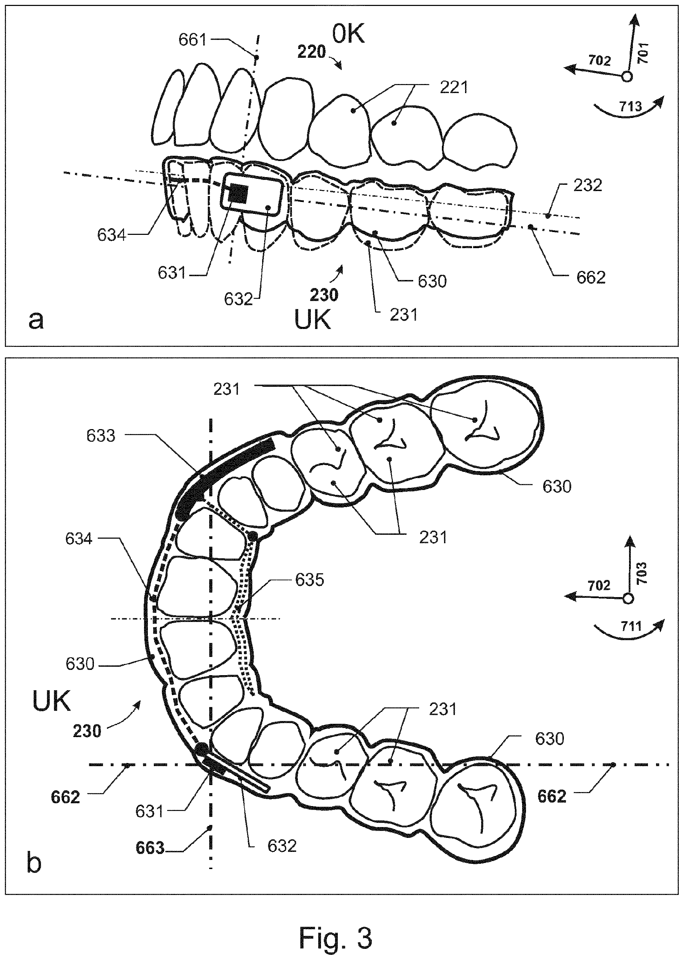

Fig. 3 zeigt oben in Fig. 3a die Seitenansicht des Gebisses mit nur im Unterkiefer eingesetztem Subsystem 30 das in der Befestigungsvorrichtung 630 integriert ist und die auf dem unteren zahnbogen 231 befestigt ist.

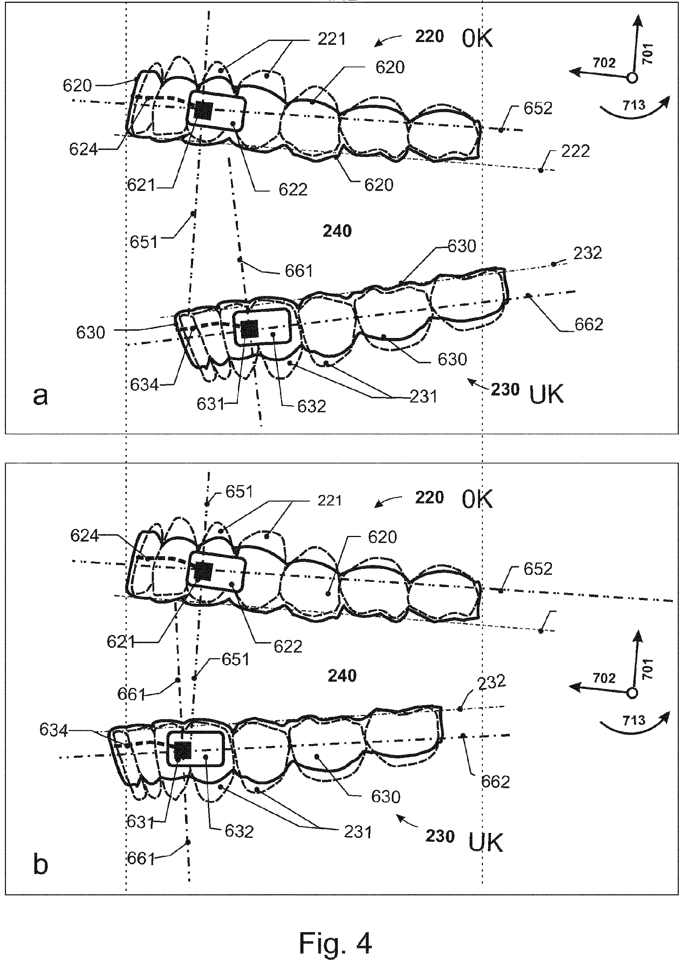

Fig. 4 zeigt Oberkiefer 220 mit Vorrichtung 620 und Sensorelement 621 sowie Unterkiefer 230 mit Vorrichtung 630 und Sensorelement 231 in zwei geöffneten Positionen, oben ist der Unterkiefer nach hinten gezogen und unten ist der Unterkiefer nach vorn geschoben.

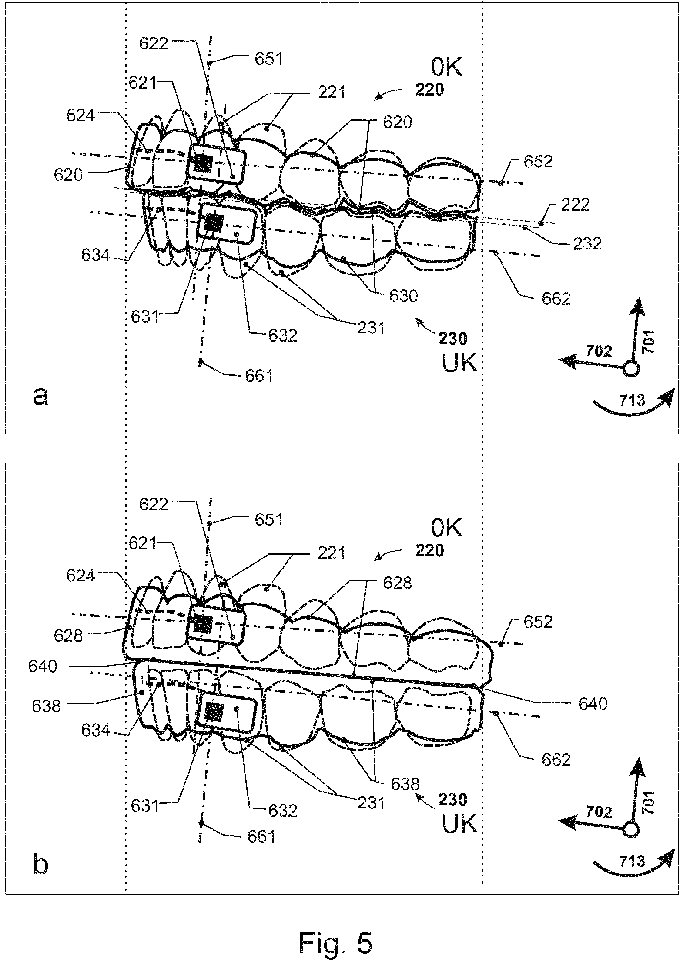

Fig. 5 zeigt messtechnisch wirksame dentale Vorrichtungen mit Oberkiefersensor 621 und Unterkiefersensor 631, die jeweils weitere Bauteile 622 und 632 aufweisen, oben in Fig. 5a eine Variante mit Retentionen Höckern und Fissuren ähnlich wie Alignerschienen und unten in Fig. 5b mit planarer Gleitfläche zwischen Oberkieferteil und Unterkieferteil.

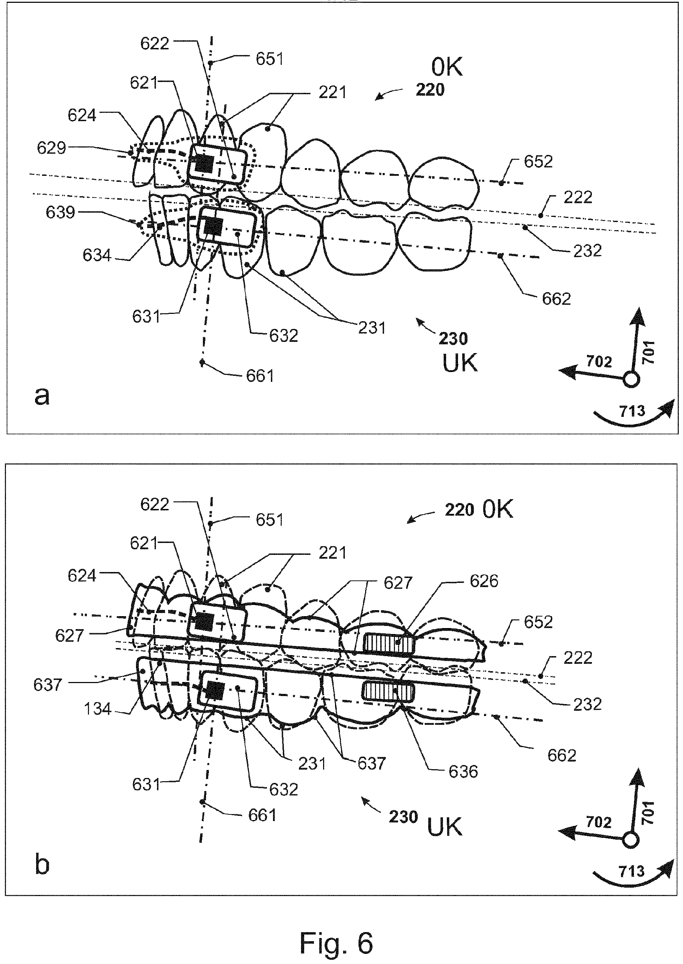

Fig. 6 zeigt weitere Varianten, um die Okklusion ungehindert messen zu können, in Fig 6a eine Version mit lokal aufgeklebten Sensorischen Elementen 621, 622 sowie 631, 632 und zudem in Fig. 6b eine Variante mit nonokklusalen Befestigungsvorrichtungen die nicht nur die Sensorelemente enthalten sondern zusätzliche Kalibriereinrichtungen 626 und 636

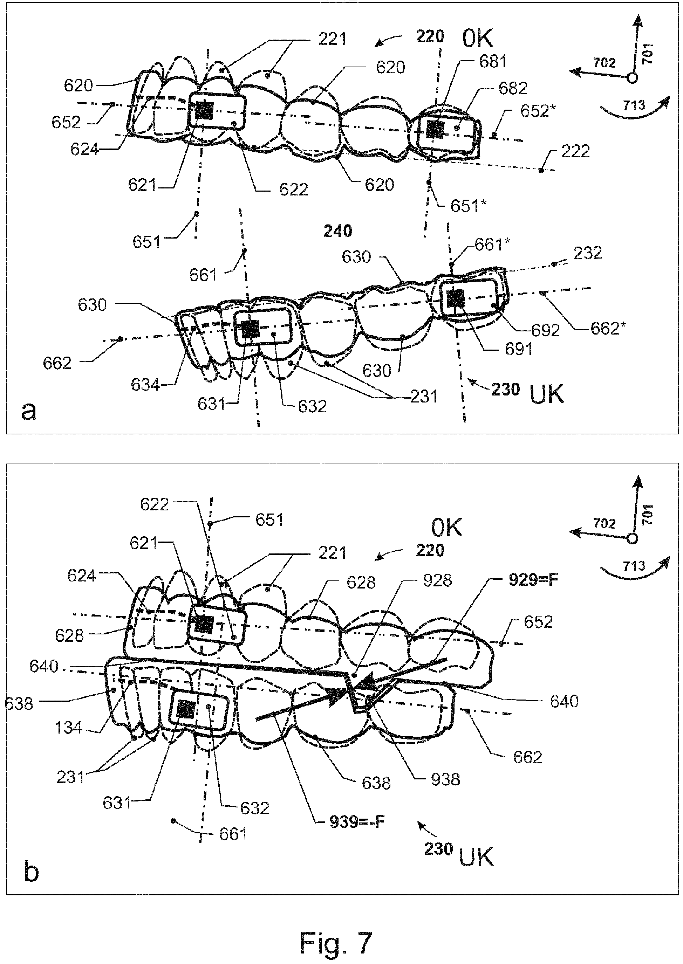

Fig. 7a zeigt weitere Varianten mit Subsystemen für den Oberkiefer und Unterkiefer mit je zwei Sensoren unterschiedlicher Empfindlichkeitsbereiche, 621 bzw. 681 sind bevorzugt für Kontaktbiss und Nahabstand bzw. weite Öffnung und Fernabstand konfiguriert bzw. umgekehrt, selbiges am Unterkiefer.
Fig. 7b zeigt eine Protrusionsschiene zur Schnarchertherapie mit integriertem Sensorsystem, Oben Subsystem 621, 622, unten Subsystem 631, 632

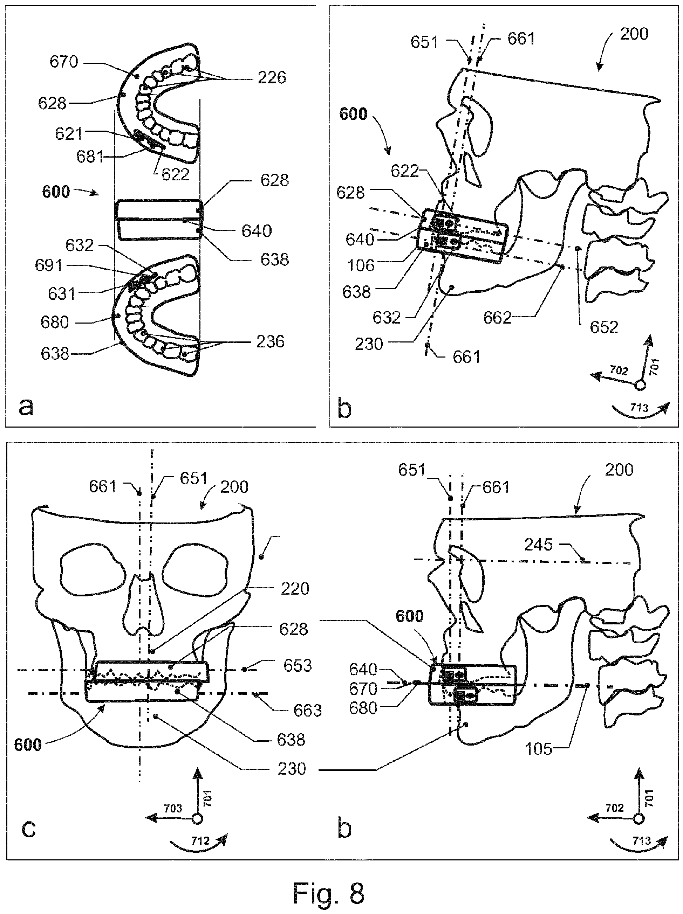

Fig. 8 zeigt das eingesetzte System zur Bewegungserfassung speziell an einem massiv ausgeführten biomechanischen System 600 mit Oberkieferteil 628 und Unterkieferteil 638. Die Besonderheit der Vorrichtung 600 ist dass sie unter Kontakt nur Translationen in der Ebene und Rotation um die Ebennormale zulässt. Dies kann zu therapeutischen Zwecken benutzt werden, dient aber auch der Kalibrierung des sensorischen Systems 100.

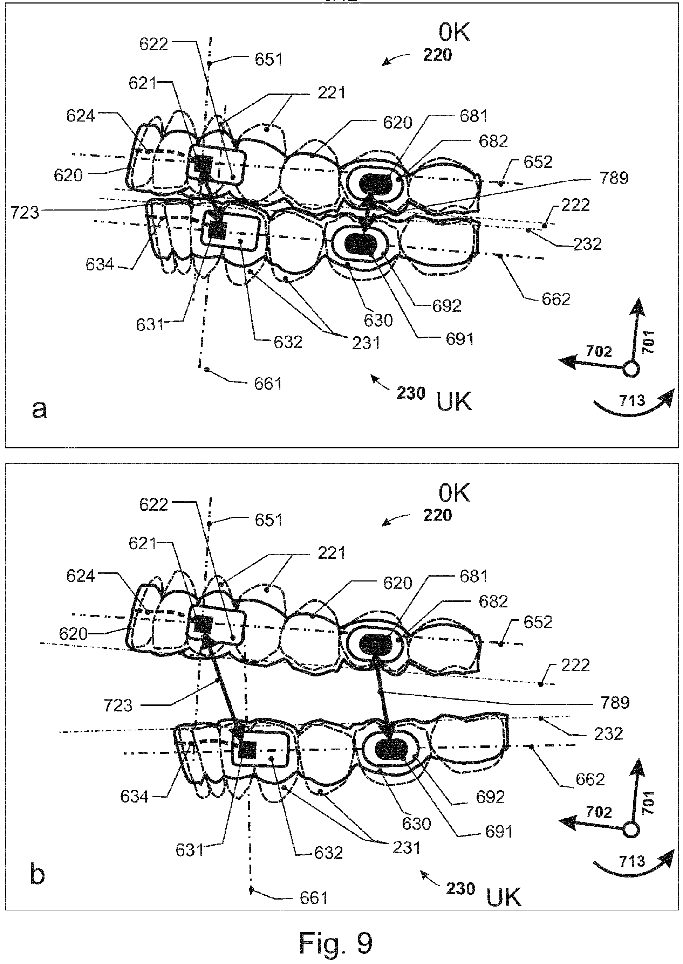

Fig. 9 zeigt die Ausbildung von Sensorgruppen zwischen Oberkiefer und Unterkiefer, wobei die sensorisch wirksame Wechselwirkung 22 und 82 zwischen dafür konfigurierten Sensorelementen aufgebaut wird, Fig. 9a oben geschlossener Mund und unten Fig. 9b geöffneter Mund mit nach hinten gerutschten Kinn, insbesondere Schnarchposition.

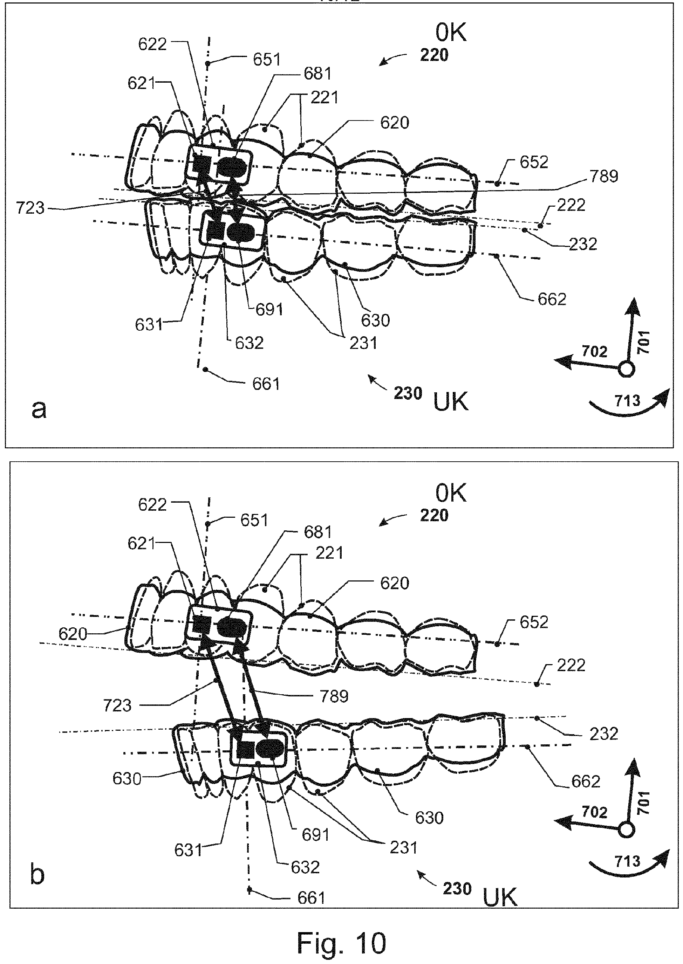

Fig 10 zeigt die direkte Integration von zwei Sensorgruppen in je ein sensorisches Bauteil 622 mit Sensoren 621 und 681 im Oberkiefer und Bauteil 632 mit Sensoren 631 und 691 im Unterkiefer. Die Sensorgruppen sind 621, 631 sowie 681, 691. Die Sensorgruppe besteht hier aus einem Sensor 621 S_motion für die Erfassung ausgedehnter Bewegungen, beispielsweise durch ein integrierendes Inertialsensorsystem kombiniert mit einem lokal genauer messenden Sensorsystems 681 S_calibartion,

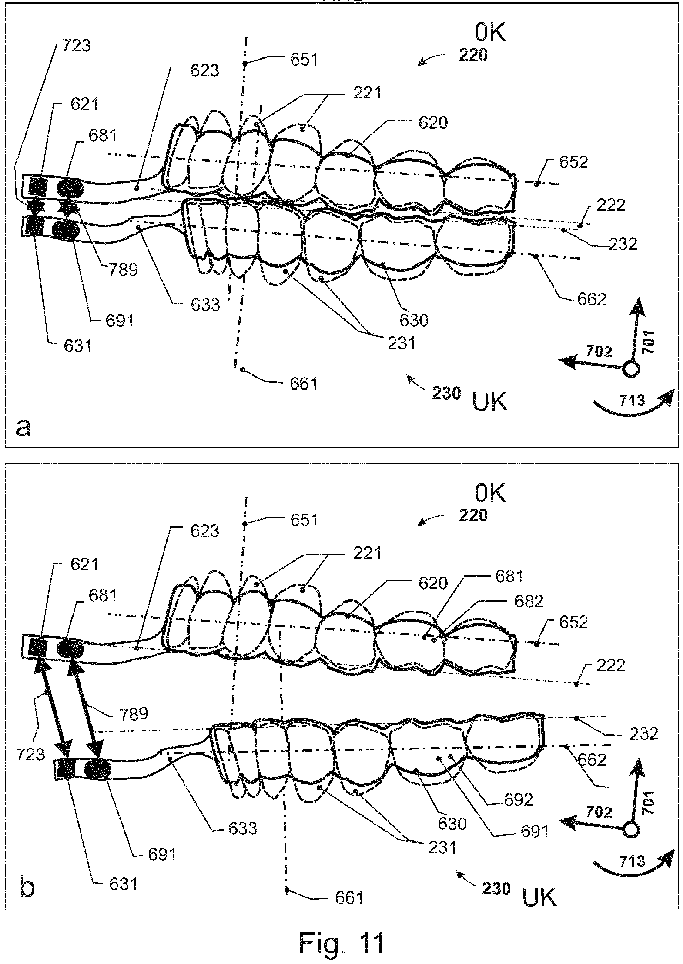

Fig 11 zeigt eine Variante für den vorwiegend kurzfristigen Einsatz mit nach vorne aus dem Mund heraus verlängerter Befestigungsvorrichtung 620 mit 623 am Oberkiefer bzw. 630 mit 633 am Unterkiefer und den an der Verlängerung angebrachten Sensorelementen 621 und 681 oben bzw. 631 und 691 unten. Die Sensorgruppen sind 621 und 631 mit Wechselwirkung 22 sowie 681 und 691 mit Wechselwirkung 82.

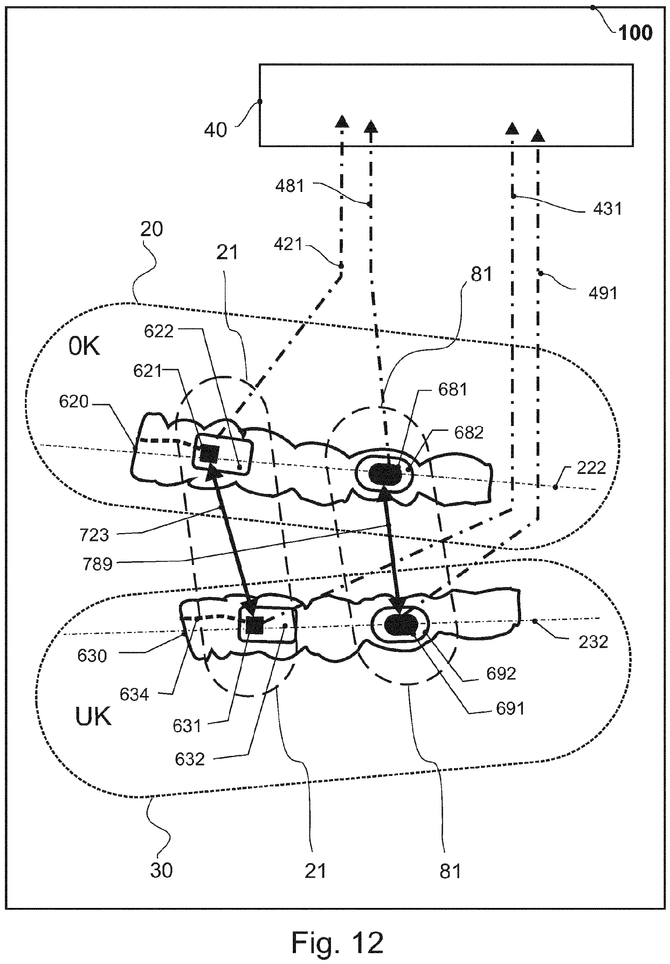

Fig. 12 zeigt das sensorisch wirkende Gesamtsystem 100 mit Subsystem 20 für den Oberkiefer sowie Subsystem 30 für den Unterkiefer. Die Sensorgruppe 21 besteht aus Sensorelementen 621 und 631. Die Sensorgruppe 81 besteht aus Sensorelementen 681 und 691. Bevorzugt ist eine Sensorgruppe besonders für die Erfassung des Nahabstandes konfiguriert und eine andere Sensorgruppe für die Erfassung des Fernabstandes und der Orientierung im Raum.

### Beschreibung einiger Komponenten und Konzepte

Das System zur Bewegungserfassung umfasst ein System zur Bewegungserfassung der Relativbewegung von Körperteilen mit mindestens zwei Subsystemen, also einem ersten Subsystem und einem zweiten Subsystem, wobei das erste Subsystem zur Befestigung an einem ersten Körperteil, zum Beispiel dem Oberkiefer oder einem starr mit dem Oberkiefer verbundenen Körperbereich wie dem Schädel, ausgebildet ist und das zweite Subsystem zur Befestigung an einem zweiten Körperteil, zum Beispiel dem Unterkiefer, ausgebildet ist, wobei das erste Körperteil und das zweite Körperteil relativ zueinander beweglich sind, und wobei das System ein Bewegungssensorsystem und ein Kalibriersensorsystem aufweist, wobei das Bewegungssensorsystem dazu ausgelegt ist, die Relativbewegung oder Relativpositionen des ersten Körperteils und des zweiten Körperteils über einen Bewegungsbereich zu erfassen, zum Beispiel, indem es Daten generiert, aus denen die Bewegungstrajektorie oder Bahnkurve der Relativbewegung des ersten Körperteils und des zweiten Körperteils erhalten werden kann oder erhalten wird, und - das Kalibriersensorsystem dazu ausgelegt ist, die Relativposition des ersten Körperteils relativ zum zweiten Körperteil zu bestimmen, wenn - zum Beispiel nur wenn und/oder immer dann wenn - das erste Körperteil und das zweite Körperteil in einem Kalibrierungsbereich relativ zueinander angeordnet sind, vorzugsweise nahe aneinander.

In einer bevorzugten Ausführung ist das Kalibriersensorsystem und/oder das Bewegungssensorsystem zur Bestimmung der Relativposition in 6-Degrees-of-Freedom (sechs Koordinaten: drei in Rotation, drei in Translation) ausgelegt. In einer anderen Ausführungsform ist es dafür ausgelegt die mittels des Kalibriersystems erhaltene aktuelle Relativposition mit einer mittels des Bewegungssensorsystems erhaltenen, vorzugsweise gleichzeitig erhaltenen, Relativposition, zu vergleichen, um einen Kalibrierungsabgleich durchzuführen.

In einer bevorzugten Ausführung ist das System so ausgelegt dass, insbesondere wenn oder nur wenn der Kalibrierungsabgleich ergibt, dass die verglichenen Relativpositionen unterschiedlich sind, das System dazu ausgebildet ist, die mittels des Kalibriersensorsystems bestimmte aktuelle Relativposition an das Bewegungssensorsystem zu übergeben, damit das Bewegungssensorsystem die aktuelle Relativposition als Ausgangspunkt für die weitere Bewegungserfassung nutzen kann.

In einer bevorzugten Ausführung werden die Positionen oder Relativpositionen immer dann überschrieben wenn das Kalibriersystem innerhalb des Kalibrierbereiches Messwerte liefert. In einem anderen Ausführungsbeispiele nur wenn die Relativposition als unterschiedlich eingestuft werden, wenn die Abweichung in mindestens einer Koordinate größer oder gleich 10 Mikrometer oder mehr als 1 Millionstel des Messbereiches, insbesondere wenn oder nur wenn der Kalibrierungsabgleich ergibt, dass die verglichenen Relativpositionen unterschiedlich sind -, auf der Basis der aktuellen Relativposition die mittels des Bewegungssensorsystems bestimmte Bahnkurve vor dem Kalibrierungsabgleich zu modifizieren, so dass die Bahnkurve durch die aktuelle Relativposition führt.

In einer besonders bevorzugten Ausführung ist das System dazu ausgebildet, dass die Modifizierung eine Glättung der Bahnkurve, insbesondere beschränkt auf einen Bereich nahe der aktuellen Relativposition, umfasst. Ferner ist es dazu ausgebildet, dass die Modifizierung die Bahnkurve an eine natürliche Bewegung anpasst.

In einer weiter entwickelten Ausführung ist das Kalibriersensorsystem und/oder das Bewegungssensorsystem zur Bestimmung der Bewegungstrajektorie oder Bahnkurve in 6-Degrees-of-Freedom (sechs Koordinaten: drei in Rotation, drei in Translation) bestimmbar. Hierzu ist das System dazu ausgelegt, mittels des Kalibriersensorsystems erhaltene Daten zur Überprüfung mittels des Bewegungssensorsystems erhaltener Daten zu nutzen. Dabei umfasst der Bewegungsbereich alle von dem ersten und dem zweiten Körperteil relativ zueinander einnehmbaren Relativpositionen, insbesondere die Relativpositionen im normalen Bewegungsablauf. Besonders bevorzugt ist der Kalibrierungsbereich eine echte Teilmenge des Bewegungsbereichs. Des Weiteren umfasst der Kalibrierungsbereich die Okklusionsstellung zwischen Ober- und Unterkiefer und/oder einen an die Okklusionsstellung angrenzenden Bereich. Besonders bevorzugt ist der Kalibrierbereich, insbesondere volumenmäßig, kleiner oder gleich einem der folgenden Prozentsätze des Bewegungsbereichs ist: 30%, 25%, 20%, 15%.

In einer besonders bevorzugten Ausführung hat Kalibriersensorsystem im Kalibrierungsbereich eine Messgenauigkeit, die größer ist als die die Messgenauigkeit des Bewegungssensorsystems, insbesondere im Bewegungsbereich und/oder im Kalibrierungsbereich. Besonders bevorzugt hat der Kalibriersensor im Kalibrierbereich eine Auflösung hat, die mindestens 5 mal so hoch ist wie die durchschnittliche Auflösung des Bewegungssensors im Bewegungsbereich.

Das erste Subsystem weist einen ersten Subsystemträger auf, der zur Befestigung an dem ersten Körperteil ausgebildet ist und/oder das zweite Subsystem weist einen zweiten Subsystemträger auf, der zur Befestigung an dem zweiten Körperteil ausgebildet ist. Dabei ist der erste Systemträger und/oder der zweite Systemträger dazu ausgebildet, als Träger für zumindest eine Komponente des Kalibriersensorsystems und/oder des Bewegungssensorsystems zu fungieren. Dabei weist das Kalibriersensorsystem zumindest einen Kalibriersensor auf.

Weitere vorteilhafte Ausgestaltungen ergeben sich aus den in den Ansprüchen und den Aspekten genannten Merkmale, die hiermit durch Bezugnahme explizit in die Beschreibung der Ausführungsbeispiele aufgenommen werden.

### System

Das System 100 umfasst bevorzugt mindestens ein Messystem für mindestens sechs Freiheitsgrade der Position und Bewegung, insbesondere drei translatorische Freiheitsgrade in der dreidimensionalen Raumposition eines Körpers und drei rotatorische Freiheitsgrade der Winkelposition eines Körpers, die man zusätzlich zur Position definieren muss, um auch die Raumorientierung des Körpers festzulegen. In den Fig. 1 sowie Fig. 3 bis Fig. 11 sind die Freiheitsgrade der Bewegung symbolisch durch ein Koordinatenkreuz markiert, das zwei orthogonale Translationsachsen 701, 702 in der Zeichenebene zeigt und eine darauf senkrecht stehende Rotationachse 713. Die Translationsachsen des Raumes sind gekennzeichnet durch die Bezugszeichen 701, 702, 703, die Rotationsachsen durch die Bezugszeichen 711, 712, 713. In Umgangssprache sind die drei Raumrichtungen 701 nach oben, 702 nach vorne und 703 nach rechts zur rechten Wange hin. Figur 1 zeigt einen Schädel, wie man ihn z.B. in einem DVT Röntgenbild im Schnittbild sieht, mit den Zähnen. Der Unterkiefer 230 artikuliert im Kiefergelenk, wobei es sich hier um ein biomechanisch muskulär geführtes Drehgleitgelenk handelt, welches Rotation und Translation erlaubt. Rotationen finden nicht nur um die transversal von links nach rechts verlaufende Achse 713 (siege Fig. 1a) statt, sondern auch um die vertikale Achse 701, z.B. bei Mahlbewegungen des Unterkiefers. So betrachtet macht es wenig Sinn, dass etliche Publikation von der Bestimmung einer Scharnierachse des Kiefergelenks sprechen, denn die Scharnierachse existiert im Kiefer ebenso wenig wie im Kniegelenk, es sei denn man baut ein mechanisches Scharnier anstelle des natürlichen Gelenks ein. Man kann allerdings anatomisch betrachtet im dreidimensionalen Röntgenbild die transversale Achse durch die beiden Krümmungszentren der Kiefergelenkskondylen einzeichnen. Man wird dann aber bei der Bewegungserfassung mit Hilfe der vorgeschlagenen Vorrichtung erkennen, dass diese anatomische Achse durch die Kondylen rechts und links nur im höchst unwahrscheinlichen Fall des reinen Drehens ohne Gleiten die Drehachse des Kiefergelenks darstellt.

Eine bevorzugt vorhandene Komponente des vorgeschlagenen Systems 100 ist das Subsystem 20 für die Bewegungserfassung des einen Körperteils, im Ausführungsbeispiel des Oberkiefers 220. Dieses Subsystem 20 erfasst auch die Bewegung des Kopfes 200 (siehe Fig. 1). Die Erfassung erfolgt mittels eines Sensor-Subsystems 621, 622, 681 (siehe Fig. 9) das am Oberkiefer befestigt ist. Zur Befestigung dient bevorzugt eine Befestigungsvorrichtung 620, die insbesondere für kurzfristige Messungen eine Verlängerung 623 nach außen aufweisen kann. Das Sensor-Subsystem kann auch direkt auf die Zähne aufgeklebt werden, wobei dann aber die Relativbewegung der Zähne zum Kiefer besonders zu berücksichtigen ist. Das Subsystem für die Bewegungserfassung des Oberkiefers wird durch ein Energieelement versorgt und kann durch ein Informationselement oder eine Speichervorrichtung die Information 421, 481 der Sensoren 221, 281 wahlweise speichern, verarbeiten und oder übertragen.

Eine weitere bevorzugt vorhandene Komponente des Systems 100 ist das Subsystem 30 für die Bewegungserfassung des anderen Körperteils, im Ausführungsbeispiel des Unterkiefers 230. Dieses Subsystem 30 bewegt sich relativ zum Subsystem des Oberkiefers OK (siehe Fig. 8a und 8b). Die Erfassung erfolgt mittels eines Sensor-Subsystems 631, 632, 691 (siehe Fig. 9) das am Oberkiefer befestigt ist. Zur Befestigung dient bevorzugt eine Befestigungsvorrichtung 630, die insbesondere für kurzfristige Messungen eine Verlängerung 633 nach außen aufweisen kann. Das Sensor-Subsystem kann auch im Unterkiefer direkt auf die Zähne aufgeklebt werden, wobei dann ebenfalls die Relativbewegung der Zähne zum Kiefer besonders zu berücksichtigen ist. Das Subsystem für die Bewegungserfassung des Unterkiefers wird ebenfalls durch ein Energieelement versorgt und kann durch ein Informationselement die Information 431, 491 der Sensoren 231, 291 wahlweise speichern, verarbeiten und oder übertragen.

Das Gesamtsystem 100 erfasst die Position und Bewegung des Oberkiefers OK und damit des Schädels 200 sowie auch die Position des Unterkiefers 230 oder alternativ direkt die Relativposition des Unterkiefers 230 gegenüber Oberkiefer 220, so dass sowohl Oberkieferposition als auch Unterkieferposition erfasst werden.

### Sensorgruppen

Das Gesamtsystem 100 hat unter anderem die Aufgabe, die sechsdimensionale Relativposition bzw. Relativbewegung, d.h. 3dim. Translation und 3dim. Rotation der beiden Körperteile zu ermitteln. Im Beispiel geht es um die sechsdimensionale Relativposition des Unterkiefers 230 in Relation zum Oberkiefer 220. Dazu besteht nicht nur die Möglichkeit die beiden Raumpositionen von Oberkiefer und Unterkiefer zu ermitteln und die Relativposition davon abzuleiten. In einer besonders bevorzugten Ausführung lassen sich aus den Bauelementen der Subsysteme 20 (OK) und 30 (UK) Sensorgruppen erstellen, die zwischen den Subsystemen 20 und 30 miteinander wechselwirken. Die Wechselwirkung besteht bevorzugt zwischen gegenüber liegenden Bauelementen wie in Fig. 12. Wechselwirkung 22 zwischen 621 und 631 und oder Wechselwirkung 82 zwischen 681 und 691. Besonders bevorzugte Wechselwirkungen sind magnetische zwischen Magneten und Magnetfeldsensoren oder optische zwischen optischen Emittern oder optischen Strukturen und optischen Sensoren. Die Elemente die eine Wechselwirkung bilden stellen Sensorgruppen 21 bzw. 81 dar (siehe Fig. 12). Die Sensorgruppen 21, 81 werden zusätzlich ausgewertet oder können auch für die Ermittlung der Relativbewegung bevorzugt ausgewertet werden. Sensorgruppen im Sinne der Messwertbildung bestehen auch dann, wenn die sensorisch wirksamen Bauelemente 621, 681 bzw. 631, 691 in einer Baugruppe zusammengefasst und räumlich eng benachbart sind (siehe Fig. 10 und Fig. 11).

### Positionssensor

Ergänzend zu der Sensorik für die Bewegungserfassung und/oder der Sensorik für die Kalibrierung kann das System einen Positionssensor umfassen (nicht explizit dargestellt), der die 6DOF-Position des Körpers, zum Beispiel anhand des Oberkiefers oder des Schädles im Realraum bestimmt. Dadurch kann die absolute Lage des beispielsweise des Kopfes genau bestimmt werden und mit anderen Ereignissen oder Auffälligkeiten, die anhand relativer Positionsdaten ermittelt werden, verknüpft werden.

### Nahabstand und Fernabstand

In einer bevorzugten Ausführung verfügt mindestens das Subsystem für den Oberkiefer 220 über eine sechsdimensionale Erfassung des Subsystems 20 im Raum und zusätzlich verfügt das Gesamtsystem 100 über mindestens zwei Sensorgruppen 21, 81 die unterschiedliche Erfassungseigenschaften hinsichtlich Abstand, Messbereich oder Auflösung haben (siehe Fig. 12). Sobald eines der Systeme ein integrierendes System ist, besteht die Herausforderung, die Integrationsfehler zu kompensieren, die sich bei komplexen und insbesondere teilweise sehr langsamen oder sehr schnellen Bewegungen ergeben. Zu diesem Zweck umfasst das System spezielle Bauelemente für den Nahabstand, die konfiguriert sind, den Abstand auf kurze Entfernung mit einer hohen Genauigkeit von bevorzugt zwei bis zweihundert Mikrometer zu messen, indem sie Sensorgruppen mit der Gegenseite bilden. Vorzugsweise sind zusätzlich andere Bauelemente 31, 91 konfiguriert, um auf größere Entfernungen im fernabstand mit besonders großem Bewegungsumfang und dennoch ausreichend genau zu messen, aber weniger genau als die Bauelemente für den Nahabstand.

Eine bevorzugte Ausführungsart umfasst eine erste Sensorgruppe 21 aus mindestens zwei messtechnisch wirksame Teilen 621, 631 für den Nahabstand und misst insbesondere die fast vollständige Annäherung des Unterkiefers an den Oberkiefer genau und ebenso die sechsdimensionale Relativbewegung und Relativposition beim kräftigen Zubeißen. Die gleiche Sensorgruppe 21 oder eine zweite Sensorgruppe 81 für den Fernabstand verfügt über messtechnisch wirksame Bauteile 81, 91 die so konfiguriert sind, dass sie bei größeren Anständen langsame und schnelle Bewegung im vollen Umfang erfassen, wenn auch mit geringerer Auflösung und Genauigkeit und möglicherweise auch mit gewissen Integrationsfehlern wie sie bei integrierenden Inertialsensoren auftreten.

Die höchste Präzision der ersten Sensorgruppe 21, 621, 631 für den Nahabstand ist bevorzugt so eingestellt, dass sie die Positionsbereiche zwischen direktem Zahnkontakt und einer leichten Öffnung von wenigen bis zu zehn Millimetern umfasst. Die höchste Präzision der zweiten Sensorgruppe 81, 681, 691 für den Fernabstand ist bevorzugt so eingestellt, dass sie die daran angrenzenden Positionsbereiche zwischen einer leichten Öffnung von wenigen bis zu zehn Millimetern und der maximalen Öffnung des Mundes bzw. der maximal zu erfassenden Entfernung des Körperteils umfasst. Durch die gemeinsame Auswertung der beiden Sensorgruppen 21, 621, 631 für den Nahabstand sowie 81, 681, 691 für den Fernabstand wird der gesamte Positions- und Bewegungsbereich mit größtmöglicher Präzision erfasst, indem der Nahabstand durch die Sensorgruppe 21, 621, 631 erfasst werden und der Fernabstand durch die Sensorgruppe 81, 681, 691. Die Zuordnung der Sensorgruppen 21, 621, 631 bzw. 81, 681, 691 auf Nahabstand und Fernabstand kann natürlich auch vertauscht werden.

### Rekalibrierung

Das System und die messtechnisch wirksamen Bauelemente und Sensorgruppen werden bevorzugt zu Beginn einer Messung kalibriert. Bei langen Tragedauern des Systems 100 im Mund sind aber darüber hinaus weitere Kalibriermaßnahmen vorgesehen, die zusammen zur insgesamt erreichten Genauigkeit führen. Das Gesamtsystem 100 verfügt hierzu über mindestens eine Vorrichtung 40 und ein Verfahren 400 zur Rekalibrierung der Relativposition im Ausführungsbeispiel zwischen Oberkiefer OK und Unterkiefer UK.

Die Vorrichtung 40 zur Rekalibrierung verfügt bevorzugt über Informationsschnittstellen 421, 481 491, 431 zu den Sensorelementen 21, 81, 91, 31 und umfasst Speicherelemente für Kalibrierwerte, Speicher für Informationen und oder Messwerte und Prozessoreinrichtungen zur Informationsverarbeitung, bevorzugt auch zur Berechnung der sechsdimensionalen Positionsdaten für Oberkiefer und Unterkiefer.

### Verfahren

Die Rekalibrierung der ermittelten Relativposition zwischen UK und OK wird durchgeführt oder alternativ durch ein Signal ausgelöst, sobald sich Unterkiefer im Bereich des Nahabstandes relativ zum Oberkiefer befindet bzw. bewegt und dabei dein Kalibrierbereich innerhalb des Nahabstandes erreicht wird, bei dem das System die Relativposition erkennt. Dann wird dieser erkannten Relativposition ein Koordinatenwert zugewiesen und damit wird die Relativbeziehung zwischen Oberkiefer und Unterkiefer rekalibriert. Für den Fall, dass die Bewegungserfassung in der vorhergehenden Zeit zu Abweichungen vom Realzustand geführt hat, werden nun die aktuell ermittelten Koordinaten des Nahabstandes eingesetzt und optional wird die vorher erzeugte Messwertreihe bevorzugt durch eine Ausgleichsrechnung korrigiert, so dass sich ein gleichmäßiger möglichst gut korrigierter Kurvenverlauf der Bewegung ergibt. Insbesondere wird durch die Rekalibrierung der Relativposition im Nahabstand erreicht, dass es zu keinen Messwertabweichungen für den Unterkiefer relativ zum Oberkiefer kommt, die bei der Widergabe der Messwerte einen Fehler in der gemessenen Relativposition bei Okklusion oder Kaubewegungen bedeuten würde. Dadurch wird erreicht, dass die Widergabe der erfassten Bewegung und Position in sechs Freiheitsgraden der Relativposition auch dann genau ist, wenn das System 100 die die gesamte Bewegung erfasst zwischendurch Messfehler erzeugt, insbesondere Integrationsfehler bei Inertialsensoren. Spätestens bei der nächsten Annäherung des Unterkiefers oder bei der nächsten Okklusion werden diese Messfehler wieder korrigiert.

### Anwendung und Nutzen

Das vorgeschlagene System aus Vorrichtungen und genau abgestimmten Verfahrensschritten ist also geeignet, die tatsächlichen Kieferbewegungen z.B. beim Essen und beim Sport oder beim Schnarchen oder beim Zähneknirschen kurzfristig, mittelfristig und auch langfristig aufzuzeichnen, insbesondere weil durch die Rekalibrierung die Addierung von Messfehlern immer wieder zurückgesetzt wird, sobald der Unterkiefer in den Nahabstand oder in Okklusion bewegt wird. All dies sind Leistungen, die frühere Systeme grundsätzlich aus technischen Gründen nicht erbringen konnten.

Die Auswertung der Bewegungserfassung hinsichtlich der Relativbewegung zwischen Unterkiefer und Oberkiefer zeigt insbesondere, dass je nach Bewegungsart und je nach Stress die Bewegungsachsen dynamisch sich sehr stark unterscheiden können, was auf sehr stark unterschiedliche Knorpelbelastung hinweist, je nachdem mit welchen Emotionen die Bewegung durchgeführt wird.

### Erfassung

Die Erfassung der Positions- und Bewegungsdaten eines Objekts, Körpers oder Körperteils bestimmt also die 6-degrees-of-Freedom Koordinaten in einem solchen Koordinatensystem mit den Koordinaten [701, 702, 703, 711, 712, 713]. Dieser Koordinaten-Datensatz wird für jeden Beobachtungszeitpunkt ermittelt und die Abfolge der Koordinaten beschreibt die erfasste Bewegung. Zusätzlich kann man noch die Größenskalierung eines Objekts angeben etc., wodurch die Datensätze entsprechend größer werden. Man kann diesen minimalistischen 6-degrees-of-Freedom Koordinatensatz auch anders schreiben, z.B. als siebendimensionaler oder achtdimensionaler Vektor statt nur mit 6 Dimensionen, wobei die Angabe dann überbestimmt ist. Und natürlich kann man die 6-Degrees-of-Freedom auch einschränken durch mechanische Führungen, dann sind aber nur entsprechend eingeschränkte Bewegungen möglich.

Im Folgenden wird zur Klarheit die minimalistische 6-Degrees-of-Freedom Schreibweise benutzt, um Positionen und Raumorientierungen von Körperteilen und Objekten eindeutig zu beschreiben, soweit diese als nicht deformierbar angenommen werden können.

### Koordinatensysteme

In Fig.1a sieht man einen Schädel mit geschlossenem Kiefer in Okklusion von vorne. Die Achse 703 zeigt von links nach rechts bezogen auf den Patientenkopf, im Bild von rechts nach links. Die Achse 701 zeigt nach oben. Fig. 1b zeigt die Koordinatenachse 703 nicht, weil sie senkrecht auf die Zeichenfläche steht. Davor sieht man von der Seite die Koordinatenachse 702, die nach vorne zeigt, im Bild von rechts nach links in der Schnittansicht von der Seite betrachtet. Die Koordinatenachse 701 zeigt auch hier nach oben. Die Rotationsachsen sind durch die Drehpfeile angedeutet. Die mathematische und physikalische Drehrichtung läuft gegen den Uhrzeigersinn. Der Unterkiefer 230 öffnet sich also in Fig. 1c im Uhrzeigersinn mit einem positiven Rotationswinkel. Betrachtet man den Kopf von der anderen Seite wie in Fig. 1c, so bleibt die positive Rotationsrichtung erhalten, weil das Koordinatensystem mit der Betrachtungsrichtung umklappt. In Fig. 7c ist eine Protrusions-Stellung gezeigt, also ein z.B. durch Muskelkraft nach vorne geschobener und leicht geöffneter Unterkiefer 230. Im Vergleich zum Oberkiefer 220 bekommen wir also eine ganz andere Position als wenn man eine Scharnierbewegung im Kiefergelenk mit hinten liegender Drehachse ansetzen würde, wie in Fig 7d gezeigt.

Die Aufgabe des vorgeschlagenen Systems kann nun darin bestehen, alle Bewegungen, schnelle Positionswechsel und auch schleichende Gleitbewegungen korrekt zu erfassen und dabei auch immer die Relativposition genau zu messen. Das Problem liegt nun darin, dass es ganz langsam schleichende Bewegungen gibt, die insbesondere von integrierenden Sensoren kaum erfasst werden würden und dann wieder plötzlich ganz schnelle Bewegungen, die den Messbereich überfordern könnten. In einer Kombination aus langsamen und ruckartigen schnellen Bewegungen ist die Relativposition bislang auch mit den modernsten 6-Degrees-of-Freedom oder 9-degrees of Freedom Sensoren nicht präzise messbar, weil deren Auflösung nicht ausreicht, um langsame Gleitbewegungen zu erkennen und korrekt zur präzisen Position bzw. Translation zu integrieren. Das liegt an der technischen Hürde, so kleine Beschleunigungen überhaupt ansatzweise präzise erfassen zu können und dennoch große Beschleunigungen messen zu können. Die verfügbaren 6-Degrees-of Freedom Sensorelemente, die es in kleinen Bausteinen mit wenigen Millimetern Kantenlänge zu kaufen gibt, haben deshalb auf absehbare Zeit große Schwierigkeiten mit der exakten Positionsbestimmung nach langsamen Bewegungen.

Das vorgeschlagene System 100 umfasst bevorzugt mindestens zwei Vorrichtungen, wovon mindestens eine an den Unterkiefer 230 angekoppelt, wobei die Unterkiefervorrichtung 630 vorwiegend im Mund, d.h. innerhalb der Wangen und Lippen liegt, und mindestens eine Vorrichtung 620 angekoppelt an den Oberkiefer 220, wobei auch hier die Vorrichtung im Mund liegt, also von den Lippen umschlossen werden kann. Dabei können Teile der Vorrichtung innerhalb des Zahnbogens 231, 221 im Zungen und Rachenraum 240 liegen oder auch außerhalb des Zahnbogens zwischen Lippen bzw. Wangen und Zahnbogen. Jede dieser Vorrichtungen 620, 630 erzeugt zu jedem Messzeitpunkt durch Sensoren 621, 631 ein Messergebnis, das in einen 6-Degree-of-Freedom Koordinatensatz transformiert werden kann und beschreibt damit die Raumlage der Messvorrichtung 620, 630. Natürlich ist nun die Frage ob die sensorischen Messungen und Berechnungen korrekt sind und ob die gemessenen 6-Degree-of-Freedom Koordinatensätze für Oberkiefer und Unterkiefer oder andere angekoppelte Körperteile gleich den physikalisch tatsächlich vorhandenen Zaumrichtungen zu diesem Zeitpunkt entsprechen. Dies ist eine Frage der korrekten Kalibrierung der Sensoren und der korrekten Integration der Beschleunigungen und der Drehraten. Diesen Aufgaben wird die vorgeschlagene Technologie besser gerecht als jemals zuvor.

Eine zu lösende Aufgabe liegt nun in der mittel- und längerfristig präzisen Erfassung der Bewegung der Körperteile und insbesondere der Relativbewegung, insbesondere weil man nur so die Relativposition des Unterkiefers beim Kauen und beim Schnarchen genau genug messen kann. Hier helfen die vergleichsweise genauen Drehratensensorelemente nicht, weil die anspruchsvolle Aufgabe in der Integration der langsamen Translationen liegt, die, insbesondere alleine, mittels Accelerometer oder Beschleunigungssensor nicht wirklich präzise gelingt. Eine Mischung aus schnellen Ruckartigen Bewegungen und langsamen kriechenden Bewegungen führt bei der speziellen Anwendung im Mund also zu schwerwiegenden Messfehlern in der Relativposition, wenn man keine weiteren Maßnahmen ergreifen würde.

Die vorgeschlagene Lösung erzeugt eine fast beliebig oft wiederholbare Kalibrierbarkeit der Relativposition der Vorrichtungen 620 ff und 630 ff insbesondere im eingesetzten Zustand, aber auch z.B. in einem Artikulator im Dentallabor. Dazu ist es von Vorteil, wenn die Messung mit Oberkiefervorrichtung 620, 628, 627 und Unterkiefervorrichtung 630, 637, 638 etc. erfolgt, weil sich dort immer wieder keine Abstände einstellen, insbesondere im Entspannungszustand des Kiefers.

Zur Kalibrierfunktion verfügt die Oberkiefervorrichtung 620, 627, 628, ff über eine Kalibriereinrichtung 626, die fest mit der Oberkiefervorrichtung verbunden ist. Alternativ sind alle Vorrichtungsbestandteile fest mit dem Zahnbogen des Oberkiefers verbunden. Die Unterkiefervorrichtung 630, 637, 638, ff verfügt über eine dazu passende Kalibriereinrichtung 636, die fest mit der Unterkiefervorrichtung verbunden ist. Alternativ sind alle Vorrichtungsbestandteile fest mit dem Zahnbogen des Unterkiefers verbunden.

Die beiden Kalibriereinrichtungen (oder Kalibriersensoren) 626 und 636 stehen sich zumindest phasenweise gegenüber, z.B. immer dann, wenn der Unterkiefer in Ruhelage entspannt ist oder immer dann, wenn der Unterkiefer in Okklusion zum Oberkiefer ist. Diese Position wird nun nicht nur durch die inertial messenden Sensoren 621, 631 erfasst, sondern zudem durch die Kalibriersensoren. Die Kalibriersensoren 626, 636 sind paarweise so ausgestattet, dass sie eine exakte Messung der Relativposition zwischen den beiden Kalibriereinrichtungen 626 und 636 erlauben. Optional können pro Vorrichtung 620, 630 bzw. pro Zahnbogen auch mehrere solche Kalibriereinrichtungen angebracht sein.

Zur Klarheit soll der Fachmann an dieser Stelle verstehen, dass die Kalibriereinrichtungen 626, 636 nicht die Aufgabe haben, die komplette Bewegungserfassung durchzuführen. Vielmehr haben sie die wichtige Aufgabe, den Positionsabgleich, also steuerungstechnisch etwas salopp gesagt eine Nullfahrt, nach einer, vorzugsweise vorgegebenen, Reihe von Integrationsschritten durchzuführen, sobald eine erfassbare Kalibrierposition relativ zwischen Oberkiefer und Unterkiefer erreicht ist.

Geeignete Kalibriereinrichtungen arbeiten paarweise zusammen, um in vergleichsweise kleinen Messbereichen genaue Relativpositionen zu ermitteln. Dazu nutzen sie eine Technologie ausgewählt aus (Magnetfeld, elektrisches Feld, optische Interferenz, Triangulation, Mustererkennung, optische Schwerpunktsbestimmung, optische Bestimmung eines Größenmerkmals, Winkelbestimmung, Positionsbestimmung) oder vergleichbar wirkende Technologien mit feiner Auflösung, um zu definieren, welche Relativposition zwischen Kalibriereinrichtung 626 und 636 aktuell wirklich vorliegt.

Mit Hilfe der Kalibrierwerte aus den Kalibriereinrichtungen 626 und 636 können die Koordinatensysteme bzw. Messergebnisse für die gesamten Vorrichtungen 620 ff und 630ff kurz, mittel und langfristig korrigiert werden.

In einer weiter entwickelten Ausführung umfassen die Sensorsysteme der Vorrichtung(en) 620 ff, 630 ff zusätzliche Schwerkraftsensoren, um insbesondere eine Kalibrierung der Vertikalrichtung im Schwerefeld der Erde vornehmen zu können. Bevorzugt ist zumindest die für den Oberkiefer vorgesehene Vorrichtung mit einem Schwerkraftsensor ausgestattet, so dass die Vertikalrichtung für den gesamten Kopf zuverlässig bestimmt werden kann.

In einer weiter entwickelten Ausführung umfassen die Sensorsysteme der Vorrichtung(en) 620 ff, 630 ff zusätzliche Magnetfeldsensoren, um insbesondere eine Kalibrierung der Nordrichtung im Erdmagnetfeld oder in einem anderen technisch erzeugten Magnetfeld vornehmen zu können. Schwerkraft- und Magnetsensoren können auch zusammen in dem Sensorsystem einer oder beider Vorrichtungen vorliegen.

### Verfahren

Das Verfahren gliedert sich zum Beispiel in zwei Teilschritte: A) Erzeugung des System und B) Rekalibrierung des Systems. Jeder dieser Teilschritte kann eine oder auch mehrere unabhängige Lösungen enthalten.
A: Erzeugung des Systems:
   - In einem ersten Schritt werden messtechnisch wirksam ausgestattete Vorrichtungen bereitgestellt, um diese an den Körperteilen bzw. an den Zahnbögen des Unterkiefers und am Zahnbogen des Oberkiefers oder alternativ am Oberkiefer oder am Kranium befestigen zu können. Daraus werden Submodule
   - Dann werden die Submodule 20 für den Oberkiefer und 30 für den Unterkiefer gefertigt, Diese werden bevorzugt mit einer Befestigungsvorrichtung am Zahnbogen befestigt, mittels 620 am Oberkiefer und mittels 630 am Unterkiefer, alternativ erfolgt die Befestigung der sensorisch wirksamen Elemente direkt am Zahn, beispielsweise mittels Klebstoff.
   - Anschließend erfolgt eine Aktivierung der sensorischen Subsysteme für den Oberkiefer und Unterkiefer durch elektrische Energie und eine Erzeugung messbarer und erfassbarer Signale durch die Elemente der Submodule
   - Bevorzugt erfolgt eine Gruppierung von sensorisch wirksamen Elementen zu Sensorgruppen 21, 81 um die Relativposition und Relativbewegung in bevorzugt 6 Koordinaten bestimmen zu können. Zusätzlich wird bevorzugt die Bewegung des Kopfes gleichbedeutend mit der Bewegung des Oberkiefers mit Hilfe des Subsystems
   - Bis zu diesem Schritt kann man alles vorbereiten und die Subsysteme 20 und 30 anschließend auf den zahnreihen montieren.
B: Rekalibrierung des Systems
   - Nun werden Bewegungen durchgeführt und die Bewegung des Oberkiefers wird direkt erfasst und die Bewegung des Unterkiefers wird ebenfalls direkt und oder indirekt über seine Relativbewegung zum Oberkiefer erfasst.
   - Im Falle der Verwendung von Integralsensoren werden die Messdaten gefiltert und integriert und auf diese weise wird eine Trajektorie in bevorzugt sechs Koordinaten berechnet.
   - Nun werden mit Hilfe der Sensorgruppe, die für den Nahabstand konfiguriert ist, die Koordinaten des Unterkiefers relativ erfasst sobald der Unterkiefer im genauen Messbereich der sensorgruppe für den nahabstand liegt.
   - Entweder automatisch oder sobald ein Signal für die Rekalibrierung vorliegt, insbesondere bei Annäherung des Unterkiefers an den Oberkiefer mit Annäherung der Zähne wird die Messung durch die Sensorgruppe für den nahabstand benutzt um insbesondere auch die Sensorik für den fernabstand zu rekalibrieren.
   - Anschließend oder zeitnah wird optional eine Korrektur der Messwerte durchgeführt, die durch die sensorgruppe für den fernabstand vor der Rekalibrierung erzeugt oder geliefert wurden, insbesondere um einen korrekten harmonischen Übergang in Ort, Geschwindigkeit und Beschleunigung zu erreichen.
   - Beim Erreichen definierter Näherungszustände wie beispielsweise dem Kontaktbiss, der eine Bewegungsumkehr erzwingt weil keine weitere Zusammenführung mehr möglich ist, werden auch die Sensoren für den nahabstand bei Bedarf kalibriert.
   - Beim Erreichen von vergleichsweise langsamen Bewegungsphasen wird optional bei Bedarf eine Rekalibrierung der Sensorgruppe für den Fernabstand durchgeführt.

Durch die Erzeugung des Systems mit Bildung einer Sensorgruppe für den Nahabstand und einer Sensorgruppe für den Fernabstand wird eine Rekalibrierung möglich gemacht.

Durch die Bewegung in den Nahabstandsbereich und weiter durch die Annäherung zum Kontakt zwischen den Zahnbögen, in Okklusion, wird die Rekalibrierung ausgelöst und damit werden Integrationsfehler in der Erfassung des Fernabstandes und in der räumlichen sechsdimensionalen Bewegung der Körper ausgeglichen.

### Weitere Ausführungsbeispiele oder Ergänzungen

Im dem vorgeschlagenen Konzept werden Oberkiefer und Unterkiefer bevorzugt mit jeweils einem mindestens 6-Degrees-of-Freedom Sensor erfasst, also ggfs. auch mit einem 9-degrees-of-Freedom Sensor, der mit Vorzug zudem die Schwerkraft und das Magnetfeld auswertet oder auswerten kann, wie bei einem Navigationssystem. Dabei kann das Messsystem im Mund direkt auf oder an die Zähne oder Implantate angekoppelt werden durch Aufkleben oder Anschrauben oder ähnlich fixierend wirkende Mittel. Oder das Messystem wird in eine Schiene oder in eine anderweitig ankoppelbare Vorrichtung eingebettet, z.B. in eine schlafmedizinische Protrusionsschiene oder eine kieferorthopädische Vorrichtung.

In einer besonders bevorzugten Ausführung umfasst das System 100 einen Magnetfeldsensor in der Sensorgruppe 21 und oder 81. Dieser erfasst ein Magnetfeld, das das Körperteil durchdringt, in oder an dem der Magnetfeldsensor angebracht ist. Im Oberkiefer 220 erfasst der Magnetfeldsensor bevorzugt entweder das Erdmagnetfeld oder ein Magnetfeld, das im umgebenden Raum aufgebaut ist, insbesondere um ein stärkeres und vergleichsweise besser zu erfassendes Magnetfeld nutzen zu können. Im Unterkiefer 230 erfasst der Magnetfeldsensor bevorzugt ein Magnetfeld, das besonders bevorzugt einen mindestens fünffach höheren Gradienten hat und eine mindestens fünffach höhere Stärke als das Erdmagnetfeld und das relativ zum Oberkiefer ortsfest ist. Bei geeigneter dreidimensionaler Gestaltung des Magnetfeldes erlaubt die bekannte Geometrie des Magnetfeldes des Magnetelements die Positionsbestimmung bzw. Bewegungserfassung bzw. Navigation des Unterkiefers 230 relativ zum Oberkiefer 220. Hierzu kann alternativ die Magnetfelderzeugung nicht im Oberkiefer 220 sondern im Unterkiefer 230 angebracht sein und damit ortsfest zum Unterkiefer 230 sein und als Gegenstück die Magnetfeldsensorik im Oberkiefer 220 angebracht sein.

In einer bevorzugten Ausführung des Systems 100 sind die Sensorgruppen 21 für die Erfassung des Nahabstandes und 81 für die Erfassung des Fernabstandes aus unterschiedlichen messtechnisch wirksamen Elementen und Sensortypen kombiniert. Die Sensorgruppe für den Nahabstand umfasst mindestens eine magnetfelderzeugende Anordnung aus permanentmagnetischem Material sowie mindestens einen Magnetfeldsensor wobei die Reichweite des definierten Magnetfeldes auf wenige Millimeter begrenzt ist. Die Sensorgruppe für den Fernabstand umfasst hingegen eine Kombination ausgewählt aus Drehratensensoren und Beschleunigungssensoren, deren Signale integriert werden.

In einer anderen bevorzugten Ausführungsform umfasst die Sensorgruppe für den Nahabstand optische Muster oder Signalelemente sowie optische Sensoren. Diese optische Sensorik arbeitet besonders bevorzugt mit der Erkennung von Mustern und Interferenzmustern, die sich bei definierten Abständen ergeben, um daraus Kalibriersignale abzuleiten.

In einer weiter entwickelten Ausführungsform umfasst die Sensorgruppe für den Nahabstand eine kurzreichweitige magnetfelderzeugende Anordnung mit Permanentmagneten und die Sensorgruppe für den Fernabstand umfasst eine langreichweitige magnetfelderzeugende Anordnung mit Permanentmagneten. Im Nahabstandsbereich wird die Messung der Relativbewegung durch die kurzreichweitige magnetfelderzeugende Anordnung mit Permanentmagneten unterstützt oder allein geleistet, im Fernabstandsbereich durch die langreichweitige magnetfelderzeugende Anordnung in Wechselwirkung mit den jeweiligen Magnetfeldsensoren auf der Gegenseite. Dabei können die permanentmagnetischen Anordnungen wahlweise auf der Seite des Unterkiefers angebracht sein oder auf der Seite des Oberkiefers. Die Anbringung der Magnetfeldsensoren erfolgt auf der gegenüberliegenden Seite und besonders bevorzugt auf der Oberkieferseite, weil dann keine Signalversorgung für den Unterkiefer erforderlich ist.

In einer weiteren bevorzugten Ausführungsform wird die Relativbewegung des Oberkiefers und damit des Schädels im Raum über Inertialsensoren durchgeführt, wobei diese mit Hilfe einer zweiten Sensorgruppe rekalibriert wird, weil ansonsten wie schon beschrieben Integrationsfehler entstehen würden, weil sehr langsame und sehr schnelle Bewegungen meist nicht korrekt erfasst und integriert werden. Die Rekalibrierung erfolgt hier nicht mittels Magnetfeldsensor sondern mittels Schwerkraftsensor, sobald sich geeignete Ruhephasen oder Bewegungsphasen einstellen, in denen die Schwerkraftmessung durchführbar ist. Anschließend wird besonders bevorzugt eine Korrektur der Bewegungserfassung durchgeführt, um harmonisch zusammenhängende Messkurven für Bewegung, Geschwindigkeit und/oder Beschleunigung zu erhalten.

Eine bevorzugte Ausführungsform umfasst eine Energieversorgungseinrichtung und Informationstransfereinrichtung. Die vorgeschlagene Vorrichtung 630 oder auch 620 umfasst hierzu bevorzugt eine Sensoreinrichtung 631, eine Prozessoreinrichtung 632, eine Energiespeichereinrichtung 633, einen Energie- und Datenkanal 634 und optional eine Energieversorgungseinrichtung 635, die mit der Energiespeichereinrichtung 633 verbunden ist. Die Bauteile sind angekoppelt, bevorzugt angeklebt oder eingebettet in eine Befestigungsvorrichtung, die individuell passend kraftschlüssig und formschlüssig am zahnbogen des Patienten oder Probanden befestigt werden kann, bevorzugt reversibel lösbar, z.B. als fest sitzende Spange oder Schiene.

Abschließend sei am Beispiel einer zweiteiligen Trainingsvorrichtung (siehe Fig. 7) die aus den Modulen 628 und 638 besteht nochmals gezeigt, wie in diesem besonderen Ausführungsfall die Ankopplung an die Körperteile Unterkiefer 230 und Oberkiefer 220 erfolgt und wie dann die Bewegungsmessung über einen kurzen oder auch langen Zeitraum durchgeführt wird. Die Trainingsvorrichtung wird beispielsweise dreimal täglich für zwanzig Minuten eingesetzt und die Kieferbewegungen sollen dabei aufgezeichnet werden.
- Die Vorrichtungen 628, 638 werden anhand von individuellen Scandaten von Zahnbögen und oder und oder individuellen dreidimensionalen Röntgenaufnahmen und oder individuellen Abformungen hergestellt, so dass die Vertiefungen 226, 236 genau zum zahnbogen passen und eine individuell angenehme aber auch feste Kopplung zwischen Vorrichtung und Zahnbogen und damit Kiefer sicherstellen.
- Die Vorrichtungen für Oberkiefer und Unterkiefer bekommen eine Funktionell geeignete Gestaltung in der zueinander zugewandten Seite 670 und 680, im Ausführungsbeispiel eine plane Gleitfläche
- Die Vorrichtungen bekommen im Oberkieferteil 620 ff die Funktionselemente 621, 622, 623, 624, 625, 626 integriert und ebenso im Unterkieferteil 630 ff die Funktionselemente 631, 632, 633, 634, 635, 636 und bei Bedarf auch 628, 638 oder andere und weitere Elemente
- Der Energiespeicher wird bevorzugt vor dem Einsetzen geladen
- Die Datenverbindung wird bevorzugt aufgebaut, es sei den Data Logger betrieb auf Speicher
- Die Vorrichtungen werden eingesetzt, so dass sie formschlüssig und kraftschlüssig verankert sind relativ zum Oberkiefer bzw. zum Unterkiefer
- Die Bewegungen werden mit Kopf und Oberkiefer sowie relativ dazu auch mit dem Unterkiefer durchgeführt
- Die Messungen laufen währenddessen und zeichnen separat die Bewegungen des Oberkiefers und damit des Kopfes auf und die Bewegungen des Unterkiefers
- Die Positionen werden bevorzugt durch einfache oder zweifache Integration der Sensorsignale ermittelt, wenn es sich um Beschleunigungssensoren oder Drehratensensoren handelt.
- Immer wenn die Relativposition in einen Kalibrierbereich der Kalibriereinrichtungen 626, 636 kommt, werden die Koordinaten der Vorrichtungen 620ff 630ff neukalibriert
- Die Bewegungskurven werden mit den neukalibrierten Werten fortgesetzt
- Die Werte vor der neukalibrierung werden bei Bedarf optional korrigiert
- Die Neukalibrierung wird so oft wie möglich wiederholt, sobald die Kalibriereinrichtungen 626, 636 in den Kalibrierbereich kommen, in dem die Kalibriersensoren exakte Relativpositionen bestimmen können

Die Neukalibrierung mit den Positionsmessungen hat den großen Vorteil, dass nun nicht mehr integriert werden muss, um die Koordinaten der Vorrichtungen 620ff und 630ff zu erhalten, nun reicht eine sehr präzise Koordinatentransformation. Der Kalibrierbereich, in dem die Kalibriereinrichtungen die Relativposition bestimmen können, kann eine definierte Relativposition zwischen Ober- und Unterkiefer sein. Diese definierte Relativposition kann eine Position sein, die im üblichen Bewegungsablauf häufig eingenommen wird, zum Beispiel die Okklusionsstellung oder eine vom Benutzer des Systems als besonders bequem empfundene Stellung von Ober- und Unterkiefer relativ zueinander. Die definierte Relativposition kann also nutzerspezifisch sein. Wird die definierte Relativposition eingenommen, kann, vorzugsweise automatisch, die Neu- oder Rekalibrierung erfolgen.

Der jeweilige Sensor und/oder die jeweilige Kalibriereinrichtung kann intraoral angeordnet sein. Die gesamte jeweilige Vorrichtung kann intraoral angeordnet sein, so dass die jeweilige Kieferbewegung, zum Beispiel während des Schlafes, nicht durch aus den Mund hervor ragende Elemente behindert wird. Dies kann die Akzeptanz des vorgeschlagenen Systems durch die Benutzer signifikant erhöhen.

### Weitere Details zu den Figuren

Fig. 1 zeigt einen schematisch dargestellten Schädel mit Zahnreihen. Das vorgeschlagene System wird für die Erfassung der Bewegungen des Kiefergelenks benutzt, wobei sich hier der Unterkiefer relativ zum Oberkiefer bewegt, weil die Muskulatur oben am Kranium ansetzt und unten am Unterkiefer. Zusätzlich erfolgen jedoch überlagerte Bewegungen des Kraniums durch die Hals und Nackenmuskulatur und des Weiteren die Bewegungen des Körpers selbst. Deshalb muss man die Relativbewegung zwischen Unterkiefer 230 und Oberkiefer 220 betrachten um die Relativbewegung der Zähne und die sechsdimensionalen Bewegungen im Kiefergelenk messen zu können.
Fig. 2 zeigt die Zahnbögen an einem vollständig aussehenden Beispiel, wobei die Weisheitszähne fehlen. Form und Größe der Zähne und der Kauflächen sind ausgesprochen individuell. Sie werden digital durch dreidimensionale Bildgebung oder durch Abformung erfasst und diese Form der Zahnbögen wird benutzt, um exakt individuell passende Befestigungselemente und Vorrichtungen 620, 630 herzustellen.
Fig. 3a zeigt die Vorrichtung 630 eingesetzt auf den Unterkieferzahnbogen 231, wobei hier der Oberkiefer 220 keine Vorrichtung 620 trägt. Diese Situation kann im System benutzt werden, wenn die Bewegung des Oberkiefers auf andere Weise erfasst wird, z.B. durch Ankopplung an den Schädel, insbesondere für den Fall eines zahnlosen Oberkiefers vor Totalrekonstruktion. Es ist aus Sicht des vorgeschlagenen Systems also gleichwertig, wenn die 6-Degree-of-Freedom Koordinaten der Bewegung des Oberkiefers indirekt aus der Bewegungsverfolgung des Kopfes gewonnen wird, solange die Koordinaten zeitabhängig präzise genug sind. Fig. 3b zeigt als Ausführungsbeispiel ein Subsystem für den Unterkiefer mit integrierendem Sensorelement 631 sowie weiteren Bauelementen 632 und eine Energiequelle 633 und eine Leitungsverbindung 634 sowie eine Sendevorrichtung 635. Alles ist in das Befestigungselement 630 integriert das in diesem Ausführungsbeispiel die Form eines gewöhnlichen Aligners hat. Die geometrische Form der z.B. schienenförmigen Vorrichtungen 620 und 630 kann den individuellen Zahnbögen angepasst sein und abgerundete Fissuren und Kauflächen umfassen, wie in Fig 5a dargestellt. Damit wird z.B. das Bewegungsverhalten beim Tragen einer Aufbissschiene simuliert und messbar gemacht. Die Bauelemente 632, 622 enthalten bevorzugt Prozessoreinrichtungen, die über eine Energiequelle oder einen Energiespeicher mit Energie versorgt werden. Optional ist ein Speicher zum Abspeichern und bereithalten von Daten in das Bauelement 632 integriert. Zudem können weitere Sensorelemente integriert sein. Die Datenausgabe erfolgt optional über ein Funksignal oder mit anderen Verfahren. Besonders bevorzugt wird die Energiequelle 635, 625 mit ihrer Antennenfunktion benutzt, um die ermittelten Bewegungsinformationen an einen Empfänger zu übertragen. Der Empfänger kann eine externe Rechnereinrichtung sein, z.B. ein Server oder einer der im System befindlichen Prozessoreinrichtungen. Die Antennenfunktion des Energieversorgungselements 635, 625 kann also Sendefunktion und auch Empfangsfunktion für Energie und auch für Daten übernehmen.
Fig. 4 zeigt unterschiedliche relative Positionen des Unterkiefers bei gleicher Instrumentierung. Sobald der Patient oder Proband den Kiefer öffnet, überlagern sich Drehungen um bis zu drei Achsen mit Verschiebungen in bis zu drei Raumrichtungen. Dabei ändern sich die 6-Degree-of-Freedom Koordinaten es Unterkiefers und meist auch des Oberkiefers, insbesondere aber die Relativposition des Unterkiefers zum Oberkiefer, die sich aus den beiden Koordinatensätzen von Oberkiefer und Unterkiefer ergibt. In dem vorgeschlagenen Konzept erzeugt der Oberkiefersensor 621 mit Prozessoreinheit 622 ein Oberkiefer-Koordinatensystem mit den Achsen 651, 652, 653 und der Unterkiefer-Sensor 631 mit Prozessoreinheit 632 erzeugt ein Koordinatensystem 661, 662, 663, das im Positionsursprung (Achsenkreuz) im Raum anders liegt und bei dem die Achsen andere Raumorientierungen haben. Im Vergleich zwischen Fig. 4a und Fig. 4b sieht man den Unterschied, dass unten in Fig. 4a. der Unterkiefer 230 deutlich weiter nach vorne in Richtung der Achse 662 geschoben ist, also liegt in Fig. 4b eine Protrusionsstellung vor.
Fig. 5a zeigt die instrumentierten Zahnreihen aus Fig. 4 in geschlossener Stellung, im Kontaktbiss, wobei hier die Befestigungsvorrichtungen als Aufbissschiene wirken und einen zusätzlichen Abstand des Unterkiefers vom Oberkiefer erzeugen. Arbeitet man hingegen mit nicht okklusal abdeckenden sondern nur seitlich haltenden Befestigungen können sich die Zahnbögen weiter annähern.
Fig. 5b zeigt eine zum biomechanischen Training dienende Vorrichtung 600 bestehend aus den Modulen 628 und 638 gezeigt. Hier entsteht die Trainingsfunktion durch die plane Gleitfläche 640 in der sich Oberkieferteil 628 und Unterkieferteil 638 gleitend berühren können. Im Kontaktfall mit feuchten Oberflächen ist die Reibung sehr gering und folglich muss die Muskulatur mangels Okklusionsretentionen die Positionsstabilisierung leisten. Dies führt zu speziellen und neurophysiologisch sehr nützlichen Trainingseffekten, die nun mithilfe der Bewegungserfassung in den Sensoren 631 und 621 durch die Prozessoreinrichtungen 632 und 622 aufgezeichnet werden können.
Fig. 6 zeigt zwei unterschiedliche nicht okklusal abdeckende Befestigungsvarianten. Für besonders langfristige Anwendungen können die Funktionselemente wie in Fig. 6a schematisch gezeigt mit z.B. Sensoreinheit 631, 621, Prozessor- und Speichereinheit 632, 622, Energiespeicher 633, 623 etc. jeweils direkt oder in der Art geklebter Bracketfixierungen an den Zähnen oder auch an Implantaten befestigt werden, so dass man keine Schiene mit individuelle ausgeformten Bereichen für die Zahnbögen benutzen muss, um zum gleichen Ergebnis zu kommen. Das hat den Vorteil, dass keine Relativbewegung zwischen befestigungsschiene und zahnbogen auftreten, allerdings kann der Zahn, der den Bewegungssensor trägt, sich anders bewegen als der Kiefer, weil der Zahn im Kiefer elastisch aufgehängt ist.
Fig. 6b zeigt sogenannte nonokklusale Attachments als seitlich haltende Spangen, die die Kauflächen frei lassen und so den natürlichen Kontakt der Zahnbögen ermöglichen. Diese werden verwendet um die ungehinderte Okklusion messen und dynamisch beobachten zu können, die die vorgeschlagenen Baugruppen seitlich lingual oder zungenseitig und/oder bukal oder backenseitig oder labial oder lippenseitig tragen, wobei die Kauflächen nicht überdeckt sind sondern frei bleiben. Solche Vorrichtungen 627,637 haben meist spangenform und werden oft individuell angepasst und dabei durch Klemmkräfte und/oder durch Haftmittel gehalten.
Fig 7a. zeigt eine vorgeschlagene Ausführung zur Genauigkeitsverbesserung. Dabei werden innerhalb eines Subsystems für den Oberkiefer oder Unterkiefer mehrere Sensorelemente 621, 681 bzw. 631, 691 pro Zahnbogen bzw. pro Körperteil eingesetzt bzw. aufgebracht und angekoppelt. Dies erfolgt in einem vorgeschlagenen Konzept, um gleichzeitig mit den beiden Sensorelementen unterschiedliche Aspekte der Bewegung optimiert erfassen zu können. In einer besonders bevorzugte Ausführungsform kann das eine Sensorelement langsame Bewegungen sehr gut verfolgen und integrieren, während der andere Sensorelement schnelle Bewegungen und hohe Beschleunigungen sehr gut erfassen kann. Die beiden Sensortypen können auch in einem Sensorbaustein integriert sein, sie können, müssen aber nicht an verschiedenen Stellen der Vorrichtung angebracht sein. In einer besonderen Ausführungsform befindet sich eines der Sensorelemente 681 weiter hinten im Backenzahnbereich, wenn es funktionell vorteilhaft ist und ein anderes Sensorelement 621 ist weiter vorne im Bereich der Eckzähne oder Schneidezähne. Diese Anordnung kann man beispielsweise verwenden, um den Nahbereich mit dem einen Sensorelement 621 besonders gut zu erfassen und den Fernbereich mit weiter Kieferöffnung mit dem anderen Sensorelement 681. Dabei kann der Abstand genutzt werden, um den geeigneten Bewegungsumfang zu erreichen oder um beispielsweise räumliche Magnetfelder sensorisch besser aufzulösen. Die Überbestimmtheit der Messung mit zwei oder mehr 6-Degree-of-Freedom Sensoren lässt sich zudem für die Fehlerkorrektur verwenden und zudem für die Ermittlung von elastischen oder bleibenden plastischen Verzerrungen in der Vorrichtung 620 ff und 630 ff.
Fig. 7b zeigt eine weitere wichtige Anwendung der Bewegungserfassung insbesondere zur Messung der nächtlichen Kieferbewegung bei Schnarchern mit und ohne Schnarcherschiene. Gezeigt ist schematisch eine mindestens zweiteilige Schnarcherschiene mit Oberkiefermodul 628 und Unterkiefermodul 238. Als Schrarcherscheine werden bevorzugt Protrusionsschienen eingesetzt, die den Unterkiefer mit der Kraft F nach vorne schieben, wobei die Schubkraft nach vorne durch mechanische Elemente wie Stangen oder Hebel oder haken oder schräge Verzahnungselemente 938, 928. Die integrierte vorgeschlagene Sensorik benutzt die Module 628 und 638 der Schnarcherschiene an Stelle der Befestigungselemente 620 und 630. Die Kombination unterschiedlicher Sensorelemente 621, 622 bzw. 631, 632 ermöglicht die genaue Erfassung der Bewegung sowohl im Nahbereich mit Kraftkontakt als auch im Fernbereich bei geöffnetem Mund. Insbesondere ermöglicht die vorgeschlagene Rekalibrierung des Sensorsystems die langfristig genaue Bewegungserfassung, indem Integrationsfehler etc. immer wieder korrigiert werden. Die Rekalibrierung der Relativerfassung der Unterkieferbewegung ermöglicht insbesondere die Korrektur langsamer Erfassungsfehler, die sich ansonsten über eine Nacht ergeben würden. Und für die Bewegung des Kopfes erfolgt die Rekalibrierung durch Abgleich mit einem erweiterten Sensorsystem mit Schwerkraftmessung, so dass auch hier die ansonsten sehr störenden Integrationsfehler korrigiert werden.
Fig. 8 zeigt eine mögliche Definition der aufeinander senkrecht stehenden räumlichen Hauptachsen für die Systeme des Oberkiefers und des Unterkiefers. Die vertikalen Hauptachsen 661 des Unterkiefers und 651 des Oberkiefers bleiben nur bei reiner Translation parallel. Das Kiefergelenk erlaubt aber im Allgemeinen freie komplexe Bewegungen, bei denen die Hauptachsen des Unterkiefers in Relation zum Oberkiefer sowohl in drei Raumrichtungen verschoben werden können als auch um drei zueinander senkrechte Drehachsen rotiert werden können. Zusammen gibt es generell sechs Freiheitsgrade der Bewegung, solange die Bewegung nicht wie im hier gezeigten Fall eingeschränkt wird. In Fig. 8c ist der Unterkiefer parallel nach rechts verschoben, erscheint im Bild von vorn also nach links versetzt. Für den Fall dass man integrierende Sensoren verwendet muss man sehr genau filtern und integrieren, um so kleine Verschiebungen präzise genug erfassen zu können. Die dentale Biomechanik und Bewegungsanalyse erfordert Genauigkeiten von ca. zehn bis fünfzig Mikrometer, sobald es um die Gestaltung korrespondierender Kauflächen geht. Vorrichtungen wie hier gezeigt können einerseits genutzt werden um sozusagen die Richtungstrennung des Messsystems zu testen. Darüber hinaus haben sie eine sehr beeindruckende therapeutische Wirkung.
Fig. 9 zeigt oben und unten die Erzeugung von Sensorpaaren 621 und 631 bzw. 681 und 682, um mit dieser Paarung die Relativposition noch genauer erfassen zu können. Das Sensorpaar nutzt eine Wechselwirkung 22, 82 zwischen den Sensorelementen, um daraus auf die Relativposition zu schließen. Fig. 9a. zeigt den Mund geöffnet, Fig. 9b. in geschlossener Position. Ein Sensorpaar besteht z.B. aus einer permanenten Magnetanordnung (als Sensorerreger) und einem Magnetfeldsensor der das räumlich strukturierte Magnetfeld der permanenten Magnetanordnung erfasst und auswertet, um damit auf eine relative Koordinatenbeziehung zwischen Oberkiefer und Unterkiefer zu schließen. Die Sensorpaare im System können unterschiedliche Genauigkeitsbereiche aufweisen und werden entsprechend der optimierten Genauigkeit verwendet, um Schwächen der anderen Sensorpaare zu kompensieren. Dadurch kann im Rahmen der vorgeschlagenen Lösung eine möglichst hochauflösende und genaue Erfassung der komplexen Bewegungen erreicht werden.
Fig. 10 zeigt ein Ausführungsbeispiel mit zwei Sensorpaaren, wobei je zwei unterschiedliche Sensoren 621, 681 bzw. 631, 691 in einem Bauelement 622 bzw. 632 integriert sind. Fig. 10a. zeigt den Mund geöffnet, Fig. 10b. in geschlossener Position. Dabei kann ein Sensorpaar beispielsweise eine magnetische Wechselwirkung erfassen und zur Auswertung geben und ein anderes kann beispielsweise eine Gravitationswechselwirkung erfassen. In einer solchen Kombination kann zusätzlich auch ein Inertialsensor integriert sein, der Beschleunigungen und oder Drehraten erfasst und integriert. Das Wirkungsoptimum der magnetischen Sensorpaare liegt häufig im Bereich der Nahentfernung während die gravimetrischen Sensorpaare oder Sensoren feststellen, wo unten ist, z.B. um die Richtung von unten nach oben während des Schlafens trotz der Kopfbewegung laufend zu erfassen. Die kurzreichweitigen Magnetfeldsensoren können besser dazu benutzt werden um bei Annäherung des Unterkiefers an den Oberkiefer oder beim Übergang zum Kontaktbiss den nahabstand genau zu messen und bei Bedarf eine Rekalibrierung insbesondere eines Inertialsensors auszulösen.
Fig. 11 zeigt ein besonderes Ausführungsbeispiel mit einer nach außen aus dem Mund hinaus ragenden Verlängerung 623 bzw. 633 der Befestigungselemente 620 bzw. 630. Dadurch kommen die Sensorelemente in den Bereich außerhalb des Mundes und der Lippen. Dies ist z.B. erforderlich wenn das Einbringen der Bauelemente Allergien oder Störungen auslösen würde oder wenn die Sensorik durch Implantate gestört wäre. Bei den nach außen geführten verlängerten Befestigungsvorrichtungen sind die Bewegungsausmaße im Sinne des Hebelgesetztes vergrößert. Die Koordinatentransformationen bei der Bewegungserfassung berücksichtigen diese Verhältnisse. Dies führt aber nur dann zu verbesserten Genauigkeiten wenn die Befestigungen steif genug sind. Nach außen führende Verlängerungen der Befestigungselemente lassen sich vor allem kurzzeitig einsetzen. Dafür haben sie den Vorteil, mehr Platz für die Energieversorgung zu bieten und eine bessere Informationsübertragung.
Fig. 12 zeigt rein schematisch das vorgeschlagene System 100 mit den Subsystemen 20 für den Oberkiefer bzw. 30 für den Unterkiefer nur mit den Befestigungsvorrichtungen 620 bzw. 630 ohne die Zahnbögen, um bessere Klarheit zu erreichen. Die gestrichelt gezeichneten Sensorpaare 21 und 81 verbinden je ein Sensorelement aus dem Subsystem des Oberkiefers mit einem Sensorelement aus dem Subsystem des Unterkiefers und werten eine Wechselwirkung aus. Mögliche Sensorsysteme arbeiten optisch mit Erfassung grafischer Muster oder magnetisch mit Erfassung permanenter Magnetfelder oder gravimetrisch mit Erfassung der Schwerkraftrichtung. Möglich sind auch Ultraschallsysteme, bei denen eine Seite Impulse aussendet und die andere sie kurz danach empfängt. Im Rahmen der vorgeschlagenen Lösung wird mindestens ein Sensorpaar ausgewertet, um die Relativbewegung und Relativposition von Oberkiefer und Unterkiefer zu erfassen. Besonders bevorzugt werden zwei unterschiedliche oder unterschiedlich konfigurierte Sensorpaare erfasst und die Signale werden ausgewertet, um aus der Kombination der Signale eine nochmals verbesserte Auflösung und/oder Genauigkeit zu erreichen. Dabei wird besonders bevorzugt eines der Sensorpaare benutzt, um eine Rekalibrierung von Sensoren, insbesondere der integrierenden Sensoren auszulösen.

Die Komponenten des Systems können für die intraorale Anwendung vorgesehen sein oder die extraorale Anwendung. Auch Mischformen sind möglich.

### Weitere beispielhafte Ausführungsformen

Im Folgenden werden mehrere Sätze von beispielhaften Ausführungsformen der Offenbarung angegeben, die das Verständnis der Erfindung erleichtern. Diese beispielhaften Ausführungsformen bzw. Sätze sind als eigenständige Offenbarung anzusehen, die ggf. zum Gegenstand von Patentansprüchen gemacht werden kann. Die beispielhaften Ausführungsformen sind auch als die weitere Offenbarung ergänzende Offenbarung anzusehen, so dass Merkmale, die in den beispielhaften Ausführungsformen beschrieben sind, auch in anderem Zusammenhang als dem beschriebenen Zusammenhang herangezogen werden können, insbesondere in Kombination mit Merkmalen aus den verbleibenden Teilen der Beschreibung. Weiterhin können die Merkmale einzelner beispielhafter Ausführungsformen auch für sich genommen betrachtet werden, also ohne Bezug zu den anderen beispielhaften Ausführungsformen.

Gemäß einer beispielhaften Ausführungsform, wird ein System (100) zur Bewegungserfassung mit oder aus mindestens zwei Vorrichtungen (620, 630) zum vorzugsweise form- und / oder kraftschlüssigen Ankoppeln an Körperteile, insbesondere an den Oberkiefer (220) bzw. Unterkiefer (230), mit jeweils Sensorkombinationen oder Inertialsensoren und Elektroniken (621, 622) bzw. ( 631, 632), die, zum Beispiel unabhängig oder vorwiegend unabhängig, die, zum Beispiel komplexe, Bewegung von Oberkiefer (220) und Unterkiefer (230) messen oder für die zugehörige Messung ausgelegt sind und einer Elektronik, insbesondere Prozessoreinrichtung, die daraus die Relativbewegung zwischen Ober- und Unterkiefer, die Relativbewegung im Kiefergelenk und/oder die Kopfbewegung ermitteln kann oder zur Ermittlung, insbesondere der jeweiligen Bewegung oder Relativbewegung, ausgelegt ist, bereitgestellt.

Gemäß einer vorteilhaften Weiterbildung der Ausführungsform dient das System zur Bewegungserfassung insbesondere am Gebiss und am Kiefergelenk, wobei mindestens eines der Subsysteme (20, 30), insbesondere für Oberkiefer oder Unterkiefer, über zumindest einen Kalibriersensor (626, 636) verfügt, wobei der Kalibriersensor in einem lokal umgrenzten Bereich der Bewegungsmöglichkeiten, insbesondere im Bereich der Okklusionsannäherung von Oberkiefer und Unterkiefer, die momentane Relativposition der beiden Vorrichtungen (620, 630) ermittelt oder in einer länger dauernden relativen Ruhephase die sechsdimensionale Raumposition und Raumorientierung der Oberkiefervorrichtung (620) ermittelt und damit eine Rekalibrierung von Teilen des Systems (100) sowie eine Korrektur voraus gegangener und zukünftiger Messungen erlaubt.

Gemäß einer weiteren vorteilhaften Weiterbildung der Ausführungsform umfasst mindestens eines der Subsysteme (20, 30) mindestens zwei unterschiedliche oder unterschiedlich konfigurierte Sensorelemente und die Sensorelemente sind so gewählt und konfiguriert, dass eines mehr für die Erfassung des Nahabstandes konfiguriert ist und ein anderes mehr für die Erfassung des Fernabstandes der funktional entscheidenden Bereiche der Körperteile, insbesondere des Zahnbogens des Unterkiefers vom Zahnbogen des Oberkiefers.

Gemäß einer weiteren vorteilhaften Weiterbildung der Ausführungsform erfasst mindestens ein Sensor des Subsystems (20) oder des Subsystems (30) eine Wechselwirkung zwischen dem Subsystemen (20,30), ausgewählt aus Magnetfeldsensor, Lichtsensor, Ultraschallsensor oder Gravitationssensor und wobei dieses Signal verwendet wird um die Messgenauigkeit anderer Sensoren zu erhöhen oder eine Rekalibrierung oder eine Korrektur im System (100) auszulösen.

Gemäß einer weiteren vorteilhaften Weiterbildung der Ausführungsform wird mindestens ein Sensorpaar aus mindestens einem Element des Subsystems (20) und mindestens einem Element des Subsystems (30) verwendet, um eine messtechnisch auswertbare Wechselwirkung zwischen den Elementen (621, 622, 631, 632, 681, 691) der Subsysteme (20,30) zu erfassen.

Gemäß einer weiteren vorteilhaften Weiterbildung der Ausführungsform ist mindestens ein Sensorpaar (21, 81) durch mindestens einen Permanentmagneten und mindestens einem Magnetfeldsensor oder mindestens einem optisch erkennbaren Muster und einem optischen Sensor oder mindestens einer Ultraschallquelle und einer Ultraschallempfänger gebildet und wobei weitere Sensorsignale, insbesondere Inertialsensoren oder Sensoren anderer Art verwendet werden um mindestens die sechsdimensionale Position von einem der Subsysteme zu erfassen.

Gemäß einer weiteren vorteilhaften Weiterbildung der Ausführungsform wird mindestens ein Gravitationssensor verwendet um die Richtung der Schwerkraft für mindestens eines der Subsysteme zumindest zeitweise oder widerholt zu bestimmen, insbesondere für das Oberkiefersubsystems (620), wobei zudem andere Sensoren verwendet werden, um die Bewegung oder die Relativbewegung des anderen Subsystems zu erfassen, insbesondere des Unterkiefersubsystems (630).

Gemäß einer weiteren vorteilhaften Weiterbildung der Ausführungsform umfasst das System die Bauteile einer Protrusionsschiene (627, 637) zur Schnarchertherapie oder zur Apnoetherapie oder einer Trainingsvorrichtung mit transversalen Trenngleichtflächen (670, 680).

Gemäß einer weiteren vorteilhaften Weiterbildung der Ausführungsform umfasst das System mindestens zwei Sensoren unterschiedlicher Empfindlichkeit für mindestens eine der Vorrichtungen (620, 630) bzw. pro Zahnbogen, wobei der eine der beiden Sensoren für vorwiegend schnelle Bewegungen konfiguriert ist und der andere für vorwiegend langsame Bewegungen.

Gemäß einer weiteren beispielhaften Ausführungsform wird ein Verfahren zur Erfassung von Positionen, Bewegungen und Relativbewegungen von Körperteilen, insbesondere von Oberkiefer und Unterkiefer mit den oberen und unteren Zahnbögen unter Verwendung des Systems nach der obigen beispielhaften Ausführungsform, unter Durchführung der Verfahrenselemente
a) Bereitstellen von Subsystemen (20, 30) mit Befestigungselementen (620, 630) und integrierten Sensoren und Elektronik (621, 622, 631, 632, 681, 691) sowie Elementen für Energieversorgung, Datenverarbeitung und Informationstransfer und Informationsspeicherung,
b) Befestigung der Subsysteme (20, 30) an den Körperteilen, insbesondere an den Zahnbögen (221, 231 des Oberkiefers (620) und Unterkiefers (630)
c) Aktivierung der Energieversorgung der Sensorsysteme in den Subsystemen und der anderen Elemente,
d) Durchführung von Bewegungen und Relativbewegungen der Körperteile, insbesondere des Unterkiefers relativ zum Oberkiefer,
e) Erfassung der Sensorisch ermittelten Daten in den Subsystemen (20, 30),
f) Zwischenspeicherung oder Transfer der erfassten Sensordaten, insbesondere der Informationen zur Bewegung des Unterkiefers und oder des Oberkiefers,
g) Ermittlung der Bewegungsdaten mindestens eines Körperteils, insbesondere mindestens des Unterkiefers oder des Oberkiefers o der der Relativbewegung zwischen Unterkiefer und Oberkiefer, bereitgestellt.

Gemäß einer weiteren vorteilhaften Weiterbildung der Ausführungsform des Verfahrens weist das Verfahren die Durchführung der Verfahrenselemente,
a) Erfassung der sensorischen Signale mindestens eines Inertialsensors des ersten Subsystems (20) und mindestens eines Inertialsensors des zweiten Subsystems (30) und Transfer dieser Informationen an eine Elektronik oder eine Software, um daraus die Daten der Relativbewegung der beiden Körperteile in bis zu 6 Freiheitsgraden der Bewegung zu ermitteln, auf.

Gemäß einer weiteren vorteilhaften Weiterbildung der Ausführungsform des Verfahrens weist das Verfahren die Durchführung der Verfahrenselemente,
a) Erfassung der sensorischen Signale mindestens eines Inertialsensors des ersten Subsystems (20) oder des zweiten Subsystems (30) und Transfer dieser Informationen an eine Elektronik oder eine Software, um daraus die Daten der Relativbewegung des Körperteils in bis zu 6 Freiheitsgraden der Bewegung zu ermitteln,
b) erfassen der Bewegung in den Bereich eines Kalibrierbereiches eines dritten Sensors oder Sensorpaares und daraufhin auslösen einer Kalibrierung oder Korrektur der Sensorkonfiguration des Inertialsensors,
auf.

Gemäß einer weiteren vorteilhaften Weiterbildung der Ausführungsform des Verfahrens weist das Verfahren die Durchführung der Verfahrenselemente,
a) Erfassung der sensorischen Signale mindestens eines ersten Sensors des Subsystems (20) oder des zweiten Subsystems (30),
b) erfassen der Bewegung in den Bereich eines Kalibrierbereiches eines dritten Sensors oder Sensorpaares und daraufhin auslösen einer Kalibrierung oder Korrektur der Sensorkonfiguration des ersten Sensors.
c) Durchführung einer Korrektur an den zuvor aufgenommenen und ermittelten Bewegungsdaten insbesondere im Sinne einer Angleichung der zuvor bis zur neuen Kalibrierung aufgezeichneten Bewegungskurven,
auf.

Gemäß einer weiteren vorteilhaften Weiterbildung der Ausführungsform des Verfahrens weist das Verfahren die Durchführung der Verfahrenselemente,
a) Erfassung der Bewegung in einem Bereich des Nahabstandes durch ein dafür konfiguriertes erstes Sensorpaar oder einen dafür konfigurierten ersten Sensor,
b) Erfassung der Bewegung in einem Bereich des Fernabstandes durch ein dafür konfiguriertes zweites Sensorpaar oder einen dafür konfigurierten zweiten Sensor,
c) Benutzung des Sensorsignals für den Nahbereich, um bei Annäherung an einen geeigneten Kalibrierbereich innerhalb des Nahbereiches einen Kalibriervorgang oder einen Korrekturvorgang auszulösen und Kalibrierwerte oder Korrekturwerte zu speichern oder zu übertragen,
auf.

Gemäß einer weiteren vorteilhaften Weiterbildung der Ausführungsform des Verfahrens weist das Verfahren die Durchführung der Verfahrenselemente,
a) Erfassung der Bewegung mindestens eines der Subsysteme (20, 30) im Raum, insbesondere der Subsysteme für Oberkiefer und / oder Unterkiefer,
b) Erfassung des Gravitationsfeldes oder eines mit dem Gravitationsfeld und dessen Richtung gekoppelten Sensorsignals,
c) Erfassung eines Kalibrierbereiches innerhalb des Bewegungsablaufes, insbesondere eines Bereiches gleichmäßiger Bewegung oder angenäherter Ruhe oder geeigneter Raumorientierung des gravimetrischen Sensors,
d) Auslösen einer Kalibrierung der Raumorientierung mindestens des dabei erfassten Subsystems, insbesondere des Oberkiefersubsystems,
auf.

Eine weiterer Satz beispielhafter Ausführungsformen, die als das Verständnis der Erfindung erleichternd angesehen werden, ist im Folgenden angegeben.

Gemäß einer beispielhaften Ausführungsform wird ein System mit oder aus mindestens zwei Vorrichtungen zum, vorzugsweise form- und/oder kraftschlüssigen, Ankoppeln an Oberkiefer bzw. Unterkiefer mit jeweils Sensorkombinationen oder Inertialsensoren (621, 631), die unabhängig die komplexe Bewegung von Oberkiefer und Unterkiefer messen, und einer Prozessoreinrichtung (622, 632), die daraus die Relativbewegung zwischen Ober- und Unterkiefer, die Relativbewegung im Kiefergelenk und/oder die Kopfbewegung ermitteln kann oder zur Ermittlung der jeweiligen (Relativ)Bewegung ausgelegt ist, bereitgestellt.

Gemäß einer weiteren vorteilhaften Weiterbildung der Ausführungsform verfügt die, bevorzugt jeweilige, Vorrichtung, insbesondere die oben (Oberkiefer) und/oder unten (Unterkiefer), über zumindest einen Kalibriersensor (626, 636), wobei der, insbesondere jeweilige, Kalibriersensor, zum Beispiel in einem lokalen Messbereich und/oder in einer vorübergehenden Bewegungsphase oder auch in einer länger dauernden relativen Ruhephase, die exakte Relativposition der beiden Vorrichtungen ermittelt und damit eine Neukalibrierung der Position, insbesondere der Relativposition zwischen Oberkiefer und Unterkiefer, erlaubt.

Gemäß einer weiteren vorteilhaften Weiterbildung der Ausführungsform weist das System die Vorrichtungen (620, 630) auf, wobei die Grenzflächen zwischen Oberkiefer und Unterkiefer nach gewünschter Funktion ausgestaltet werden und sich dabei von einer normalen Aufbissschiene oder einem normalen Aligner unterscheiden.

Gemäß einer weiteren vorteilhaften Weiterbildung der Ausführungsform umfasst das System die Bauteile einer Protrusionsschiene (627, 637).

Gemäß einer weiteren vorteilhaften Weiterbildung der Ausführungsform umfasst das System die Bauteile (628, 638) einer Trainingsvorrichtung mit transversalen Trenngleichtflächen (670, 680).

Gemäß einer weiteren vorteilhaften Weiterbildung der Ausführungsform umfasst das System mit mindestens zwei Sensoren unterschiedlicher Empfindlichkeit für mindestens eine der Vorrichtungen (620, 630) bzw. pro Zahnbogen kombiniert mit Anpassung des einen Sensors für schnelle Bewegungen und des anderen Sensors für langsame Bewegungen.

Gemäß einer weiteren beispielhaften Ausführungsform wird ein System oder Verfahren zur Messung von Bewegungen und Relativbewegungen bereitgestellt, vorzugsweise mittels das Systems der obigen beispielhaften Ausführungsform, wobei oben (Oberkiefer) und unten (Unterkiefer) Vorrichtungen (620, 630) mit Inertialsensoren eingesetzt werden, die eine Bewegungsmessung ermöglichen und darauf aufbauend eine Bestimmung von Relativpositionen durch Integration von Beschleunigungen oder Geschwindigkeiten.

Gemäß einer weiteren vorteilhaften Weiterbildung der obigen Ausführungsform bestimmen die Sensoren die Bewegungen in mindestens drei Translationskoordinaten und drei Rotationskoordinaten, also 6-Degrees-of-Freedom umfassende Koordinatensysteme der Vorrichtungen, bevorzugt indem sie Sensorsignale von Beschleunigungsmessern und/oder Drehratenmessern addieren und/oder integrieren.

Gemäß einer weiteren vorteilhaften Weiterbildung der obigen Ausführungsform werden im Bewegungsablauf zufällig oder absichtlich Kalibrierbereiche in der Relativposition vorübergehend als Körperposition eingestellt und wobei in diesen Bewegungsbereichen eine exakte Messung der Relativposition erfolgt, bevorzugt mit Hilfe der Kalibriereinrichtungen (626, 636).

Gemäß einer weiteren vorteilhaften Weiterbildung der obigen Ausführungsform erfolgt anschließend, insbesondere nach der Messung der exakten Relativposition, eine der Neukalibrierung entsprechende Korrektur der Bahnkurven und Positionen und Orientierungen.

### Bezugszeichenliste

- 1 - 8: Kennzeichnung der Zahnpositionen im Zahnbogen, individuell oft unvollständig

- 20: Subsystem mit Sensoren und Elektronik für den Oberkiefer
- 21: Erstes Sensorpaar
- 22: Wechselwirkung zwischen Sensorpaar 21
- 30: Subsystem mit Sensoren und Elektronik für den Unterkiefer
- 81: Zweites Sensorpaar
- 82: Wechselwirkung zwischen Sensorpaar 81
- 100: Gesamtsystem

- 200: Kopf bzw. Schädel
- 220: Oberkiefer
- 221: Zahnbogen des Oberkiefers
- 222: angelegte Kauebene des Oberkiefers in einer Näherung als plane Fläche
- 226: Vertiefungen für bzw. Abdrücke der Zähne des Oberkiefers
- 230: Unterkiefer
- 231: Zahnbogen des Unterkiefers
- 232: angelegte Kauebene des Unterkiefers in einer Näherung als plane Fläche
- 236: Vertiefungen für bzw. Abdrücke der Zähne des Unterkiefers
- 240: Mundinnenraum bei geöffnetem Mund
- 270: Kondylen des Kiefergelenks
- 272: Gelenkfläche am Oberkiefer
- 273: Gelenkfläche am Unterkiefer

- 400: Verfahren

- 620: Oberkiefer-Vorrichtung, z.B. Schienenförmig, individuell passend zum Zahnbogen 221
- 621: 6-Degrees-of-Freedom fähiger Bewegungssensor im Oberkieferteil
- 622: Prozessoreinrichtung mit Speichereinheit für Daten im Oberkiefer
- 623: Energiespeicher im Oberkiefer
- 624: Energie und Datenleitung im Oberkiefer
- 625: Energie und Datenantenne im Oberkiefer
- 626: Kalibriereinrichtung im Oberkiefer
- 627: Nonokklusales Attachment, lässt Okklusionsflächen des Oberkiefers frei
- 628: Trainingsvorrichtung Oberkieferteil, mit Gleitfläche 640

- 630: Unterkiefer-Vorrichtung, z.B. Schienenförmig, individuell passend zum Zahnbogen 221
- 631: 6-Degrees-of-Freedom fähiger Bewegungssensor im Unterkieferteil
- 632: Prozessoreinrichtung mit Speichereinheit für Daten im Unterkiefer
- 633: Energiespeicher im Unterkiefer
- 634: Energie und Datenleitung im Unterkiefer
- 635: Energie und Datenantenne im Unterkiefer
- 636: Kalibriereinrichtung im Unterkiefer
- 637: Nonokklusales Attachment, lässt Okklusionsflächen des Unterkiefers frei
- 638: Trainingsvorrichtung Unterkieferteil, mit Gleitfläche 640
- 651: Koordinatenachse Oberkiefersystem nach oben
- 652: Koordinatenachse Oberkiefersystem nach vorn
- 653: Koordinatenachse Oberkiefersystem nach rechts
- 661: Koordinatenachse Unterkiefersystem nach oben
- 662: Koordinatenachse Unterkiefersystem nach vorn
- 663: Koordinatenachse Unterkiefersystem nach rechts
- 670: Gleitfläche am Oberkieferteil 628
- 680: Gleitfläche am Unterkieferteil 638

- 701: Koordinatenachse nach oben zum Scheitel
- 702: Koordinatenachse nach vorne zur Mundöffnung
- 703: Koordinatenachse nach rechts zur rechten Wange bezogen auf den Kopf
- 711: Drechachse vertikal oben unten parallel zu 701
- 712: Drechachse von hinten nach vorne liegend parallel zu 702
- 711: Drechachse von links nach rechts laufend parallel zu 703

- 928: Mechanisches Element zum Erzeugen einer Kraftwirkung am Oberkiefer
- 929: Zwangskraft F am Oberkiefer
- 938: Mechanisches Element zum Erzeugen einer Kraftwirkung am Unterkiefer
- 939: Zwangskraft am Unterkiefer

### Glossar

| | |
|---|---|
| 6DOF | 6-Degrees-of-Freedom |
| 6-Degrees-of-Freedom | Sechs mechanische Freiheitsgrade der Bewegung, eine Positionsdefinition oder Translation in drei Raumrichtungen und überlagert eine Rotation um drei Raumwinkel um die Hauptachsen. |
| Accelerometer | Beschleunigungsmesser, der nicht Geschwindigkeit oder Position sondern Beschleunigungskräfte und folglich muss auf dieser Basis zweifach integriert werden, um die Position zu erhalten |
| Bewegungsmessung | Abfolge von Aufzeichnungen über einen Zeitraum, in dem Bewegungen der erfassten Objekte bzw. Körperteile und Vorrichtungen stattfinden |
| Bukal | zur Wange hin |
| Befestigungselement | Mechanisch verschlüsseltes form- und kraftschlüssiges Element zur Ankopplung eines Kopplungselements an ein Objekt, eine Halterung oder ein Körperteil, mit mindestens einer mechanischen Schnittstelle zur mechanisch reversibel lösbar fixierten Ankopplung eines Kopplungselements |
| Drehratensensor | Sensor der die Drehgeschwindigkeit misst, meist unter Ausnutzung der durch Rotation verursachen Kräfte, um die Winkelposition zu bestimmen, muss also einmal über die Drehrate integriert werden |
| Körperteil | Objekt, dessen Bewegung gemessen werden soll, z.B. Unterkiefer oder Schädel mit Oberkiefer |
| Kopplungselement | Mechanisch verschlüsseltes form- und kraftschlüssiges Verbindungselement zur reversibel lösbaren und wiederherstellbaren Kopplung eines Messsystems oder eines Markerkörpers an den Befestigungselementen |
| Labial | zur Lippe hin |
| Lingual | zur Zunge hin |
| Nonokklusal | Die Kauflächen nicht bedeckend, so dass man ungehindert zubeissen kann |
| Prozessoreinheit | datentechnische Vorrichtung, die Rechnerelemente, Datenspeicher, Softwarespeicher, Arbeitsspeicher, Datenkanäle und Energieversorgungselemente sowie meist auch Kühlelemente umfasst. |
| Rechts | bezogen auf den Kopf zur rechten Wange hin, im Bild von vorne gesehen also nach lins gezeichnet |
| Sechsdimensional | im Allgemeinen drei Dimensionen der Translation und drei Dimensionen der Rotation umfassend, entspricht sechs physikalischen Freiheitsgraden der Bewegung |
| Wechselwirkung | physikalisch oder elektronisch auswertbare Wechselwirkung zwischen einer Ursache und einem Sensor oder einem Sender und einem Empfänger, wobei optionale beide Seiten senden und empfangen können, z.B. Magnetfelderzeuger und Magnetfeldsensor |
| Zeitpunkt | bevorzugt kurzes Mess- und Aufzeichnungsintervall, für den der Messwert ermittelt wird, endliche Dauer, also etwas unscharf |

## Patentansprüche

1. System (100) zur Bewegungserfassung der Relativbewegung von Körperteilen mit mindestens zwei Subsystemen (20, 30), also einem ersten Subsystem und einem zweiten Subsystem, wobei das erste Subsystem zur Befestigung an einem ersten Körperteil, nämlich dem Oberkiefer oder einem starr mit dem Oberkiefer verbundenen Körperbereich wie dem Schädel, ausgebildet ist und das zweite Subsystem zur Befestigung an einem zweiten Körperteil, nämlich dem Unterkiefer, ausgebildet ist, wobei das erste Körperteil und das zweite Körperteil relativ zueinander beweglich sind, und wobei das System ein Bewegungssensorsystem (621, 681, 631, 691) und ein Kalibriersensorsystem (626, 636) aufweist, wobei das Bewegungssensorsystem und das Kalibriersensorsystem unterschiedliche Sensorsysteme sind
wobei das Bewegungssensorsystem zumindest einen Bewegungssensor aufweist und wobei das Kalibriersensorsystem zumindest einen Kalibriersensor aufweist,
wobei der Kalibriersensor und der Bewegungssensor Teil verschiedener Subsysteme sind, wobei
- das Bewegungssensorsystem dazu ausgelegt ist, die Relativbewegung oder Relativpositionen des ersten Körperteils und des zweiten Körperteils über einen Bewegungsbereich zu erfassen, zum Beispiel, indem es Daten generiert, aus denen die Bewegungstrajektorie oder Bahnkurve der Relativbewegung des ersten Körperteils und des zweiten Körperteils erhalten werden kann oder erhalten wird, und
- das Kalibriersensorsystem dazu ausgelegt ist, die Relativposition des ersten Körperteils relativ zum zweiten Körperteil zu bestimmen, wenn - zum Beispiel nur wenn und/oder immer dann wenn - das erste Körperteil und das zweite Körperteil in einem Kalibrierungsbereich relativ zueinander angeordnet sind, wobei optional der Kalibrierungsbereich die Okklusionsstellung zwischen Ober- und Unterkiefer umfasst und/oder einen an die Okklusionsstellung angrenzenden Bereich umfasst,
wobei das erste Subsystem einen ersten Subsystemträger aufweist, der zur Befestigung an dem ersten Körperteil ausgebildet ist und/oder
wobei das zweite Subsystem einen zweiten Subsystemträger aufweist, der zur Befestigung an dem zweiten Körperteil ausgebildet ist und
wobei der erste Subsystemträger und/oder der zweite Subsystemträger dazu ausgebildet ist, als Träger für zumindest eine Komponente des Kalibriersensorsystems und des Bewegungssensorsystems zu fungieren.

2. System nach Anspruch 1, wobei
a) das Kalibriersensorsystem und/oder das Bewegungssensorsystem zur Bestimmung der Relativposition in 6-Degrees-of-Freedom (sechs Koordinaten: drei in Rotation, drei in Translation) ausgelegt ist, und/oder wobei
b) das System dazu ausgebildet ist, die mittels des Kalibriersensorsystems erhaltene aktuelle Relativposition mit einer mittels des Bewegungssensorsystems erhaltenen, vorzugsweise gleichzeitig erhaltenen, Relativposition, zu vergleichen, um einen Kalibrierungsabgleich durchzuführen, wobei bevorzugt
b1), insbesondere wenn oder nur wenn der Kalibrierungsabgleich ergibt, dass die verglichenen Relativpositionen unterschiedlich sind, das System dazu ausgebildet ist, die mittels des Kalibriersensorsystems bestimmte aktuelle Relativposition an das Bewegungssensorsystem zu übergeben, damit das Bewegungssensorsystem die aktuelle Relativposition als Ausgangspunkt für die weitere Bewegungserfassung nutzen kann; und/oder wobei
b2) das System dazu ausgebildet ist - insbesondere wenn oder nur wenn der Kalibrierungsabgleich ergibt, dass die verglichenen Relativpositionen unterschiedlich sind -, auf der Basis der aktuellen Relativposition die mittels des Bewegungssensorsystems bestimmte Bahnkurve vor dem Kalibrierungsabgleich zu modifizieren, so dass die Bahnkurve durch die aktuelle Relativposition führt, und/oder wobei
b3) das System dazu ausgebildet ist, dass die Modifizierung eine Glättung der Bahnkurve, insbesondere beschränkt auf einen Bereich nahe der aktuellen Relativposition, umfasst und/oder wobei
b4) das System dazu ausgebildet ist, dass die Modifizierung die Bahnkurve an eine natürliche Bewegung anpasst, und/oder wobei
c) das Kalibriersensorsystem und/oder das Bewegungssensorsystem zur Bestimmung der Bewegungstrajektorie oder Bahnkurve in 6-Degrees-of-Freedom (sechs Koordinaten: drei in Rotation, drei in Translation) ausgelegt ist.

3. System nach mindestens einem beliebig ausgewählten vorhergehenden Anspruch oder einer beliebig ausgewählten Mehrzahl der vorhergehenden Ansprüche,
wobei das System dazu ausgelegt ist, mittels des Kalibriersensorsystems erhaltene Daten zur Überprüfung von mittels des Bewegungssensorsystems erhaltener Daten zu nutzen.

4. System nach mindestens einem beliebig ausgewählten vorhergehenden Anspruch oder einer beliebig ausgewählten Mehrzahl der vorhergehenden Ansprüche,
wobei der Bewegungsbereich alle von dem ersten und dem zweiten Körperteil relativ zueinander einnehmbaren Relativpositionen, insbesondere die Relativpositionen im normalen Bewegungsablauf, umfasst, und/oder
wobei der Kalibrierungsbereich eine echte Teilmenge des Bewegungsbereichs ist und/oder wobei der Kalibrierungsbereich kleiner oder gleich einem der folgenden Prozentsätze des Bewegungsbereichs ist: 30%, 25%, 20%, 15%.

5. System nach mindestens einem beliebig ausgewählten vorhergehenden Anspruch oder einer beliebig ausgewählten Mehrzahl der vorhergehenden Ansprüche,
wobei das Kalibriersensorsystem im Kalibrierungsbereich eine Messgenauigkeit hat, die größer ist als die Messgenauigkeit des Bewegungssensorsystems im Bewegungsbereich und im Kalibrierungsbereich und/oder
wobei das Kalibriersensorsystem im Kalibrierungsbereich eine Auflösung hat, die mindestens 2 mal so hoch ist wie die durchschnittliche Auflösung des Bewegungssensorsystems im Bewegungsbereich.

6. System nach mindestens einem beliebig ausgewählten vorhergehenden Anspruch oder einer beliebig ausgewählten Mehrzahl der vorhergehenden Ansprüche,
wobei der Kalibriersensor zum Beispiel ein Magnetfeldsensor ist, wobei das Kalibriersensorsystem optional zumindest einen Kalibriersensorerreger aufweist, der dazu ausgelegt ist, ein Sensorsignal im Kalibriersensor zu erzeugen, wobei der Kalibriersensorerreger zum Beispiel ein Magnet ist, insbesondere mit einem anisotropen Magnetfeld abgestimmt auf den Magnetfeldsensor.

7. System nach mindestens einem beliebig ausgewählten vorhergehenden Anspruch oder einer beliebig ausgewählten Mehrzahl der vorhergehenden Ansprüche,
wobei der Kalibriersensor Teil des ersten Subsystems oder des zweiten Subsystems ist und wobei der Kalibriersensorerreger Teil des anderen Subsystem ist als der Kalibriersensor.

8. System nach mindestens einem beliebig ausgewählten vorhergehenden Anspruch oder einer beliebig ausgewählten Mehrzahl der vorhergehenden Ansprüche,
wobei das Kalibriersensorsystem sensorerregerfrei ist.

9. System nach mindestens einem beliebig ausgewählten vorhergehenden Anspruch oder einer beliebig ausgewählten Mehrzahl der vorhergehenden Ansprüche,
wobei
a) wobei der Bewegungssensor zum Beispiel ein Inertialsensor oder ein optischer Sensor ist, und/oder
b) das Bewegungssensorsystem zumindest einen Bewegungssensorerreger aufweist, der dazu ausgelegt ist, ein Sensorsignal im Bewegungssensor zu erzeugen, wobei der Bewegungssensorerreger zum Beispiel einer der folgenden Erreger ist: Strahlungsquelle, insbesondere eine auf den optischen Sensor abgestimmte Strahlungsquelle, und/oder
c) der Bewegungssensor Teil des ersten Subsystems oder des zweiten Subsystems ist, und/oder
d) der Bewegungssensorerreger Teil des anderen Subsystem ist als der Bewegungssensor.

10. System nach mindestens einem beliebig ausgewählten vorhergehenden Anspruch oder einer beliebig ausgewählten Mehrzahl der vorhergehenden Ansprüche,
wobei das Bewegungssensorsystem sensorerregerfrei ist.

11. System nach mindestens einem beliebig ausgewählten vorhergehenden Anspruch oder einer beliebig ausgewählten Mehrzahl der vorhergehenden Ansprüche,
wobei der Kalibriersensor und der Bewegungssensor Teil des gleichen Subsystems sind und vorzugsweise auf einem gemeinsamen Subsystemträger befestigt sind.

12. System nach mindestens einem beliebig ausgewählten vorhergehenden Anspruch oder einer beliebig ausgewählten Mehrzahl der vorhergehenden Ansprüche,
a) umfassend eine Mehrzahl von Kalibriersensoren, von denen ein erster Kalibriersensor Teil des ersten Subsystems und ein zweiter Kalibriersensor Teil des zweiten Subsystems ist, und/oder
b) umfassend eine Mehrzahl von Bewegungssensoren, von denen ein erster Bewegungssensor Teil des ersten Subsystems und ein zweiter Bewegungssensor Teil des zweiten Subsystems ist, und/oder
c) wobei der Bewegungssensor und/oder der Kalibriersensor ein Integralsensor ist, und/oder
d) wobei der Kalibriersensor und/oder der Bewegungssensor ein kontakt- oder berührungslos arbeitender Sensor ist oder wobei der Kalibriersensor und/oder der Bewegungssensor ein Kontakt- oder Berührungssensor ist, und/oder
e) wobei das System eine Prozessoreinrichtung, insbesondere einen Mikroprozessor, umfasst, der dazu ausbildet ist:
- das Bewegungssensorsystem und/oder das Kalibriersensorsystem zu steuern, und/oder
- den Vergleich der Relativpositionen durchzuführen, und/oder
f) wobei das System eine elektrische Leistungsquelle, vorzugsweise eine Batterie, aufweist, und/oder
g) wobei das System eine Speichervorrichtung, insbesondere einen Datenspeicher aufweist, wobei
g1) die Speichervorrichtung optional zur Zwischenspeicherung von Daten des Bewegungssensorsystems ausgelegt ist, und/oder
g2) das System so ausgelegt ist, dass es in der Speichervorrichtung gespeicherte Daten nach dem Kalibrierungsabgleich löscht, mit Vorzug nach erfolgter Korrektur bzw. dem Angleich der Daten des Bewegungssensorsystems, zum Beispiel der Bahnkurve, an die aktuelle Relativposition und/oder vor dem nächsten Kalibrierungsabgleich, und/oder wobei
h) das System ein Raumpositionssensorsystem umfasst, das dazu ausgebildet ist, die absolute Position des ersten Körperteils oder des zweiten Körperteils im Raum zu ermitteln, insbesondere in 6-Degrees-of-Freedom, wobei optional
h1) das Raumpositionssensorsystem ein sensorerregerloses Sensorsystem ist, und/oder
h2) das Raumpositionssensorsystem einen Raumpositionssensor aufweist, und/oder wobei
i) das System ein Orientierungssensorsystem umfasst, das dazu ausgebildet ist, die Orientierung des ersten Körperteils oder des zweiten Körperteils im Raum zu ermitteln, wobei das Orientierungssensorsystem optional einen Orientierungssensor, zum Beispiel einen Schwerkraftsensor umfasst.

13. System nach mindestens einem beliebig ausgewählten vorhergehenden Anspruch oder einer beliebig ausgewählten Mehrzahl der vorhergehenden Ansprüche,
wobei eine beliebige Komponente, eine beliebig ausgewählte Mehrzahl von Komponenten oder alle der folgenden Komponenten des Systems dazu ausgebildet sind, im Betrieb des Systems intraoral angeordnet zu sein:
- Bewegungssensor(en),
- Kalibriersensor(en),
- Raumpositionssensor,
- Schwerkraftsensor,
- Prozessoreinrichtung,
- elektrische Leistungsquelle,
- Speichervorrichtung, und
wobei optional eine beliebige Komponente, eine beliebig ausgewählte Mehrzahl von Komponenten dazu ausgebildet ist, im Betrieb des Systems extraoral angeordnet zu sein:
- Bewegungssensor(en),
- Kalibriersensor(en),
- Raumpositionssensor,
- Schwerkraftsensor,
- Prozessoreinrichtung,
- elektrische Leistungsquelle,
- Speichervorrichtung.

## Claims

1. System (100) for detecting the relative movement of body parts having at least two sub- systems (20, 30), i.e. a first sub-system and a second sub-system, wherein the first sub-system is designed for attachment to a first body part, namely the upper jaw or a body area fixedly connected to the upper jaw such as the skull, and the second sub-system is designed for attachment to a second body part, namely the lower jaw, wherein the first body part and the second body part can be moved relative to one another, and wherein the system has a motion sensor system (621, 681, 631, 691) and a calibration sensor system (626, 636), wherein the motion sensor system and the calibration sensor system are different sensor systems
wherein the motion sensor system has at least one motion sensor and wherein the calibration sensor system has at least one calibration sensor,
wherein the calibration sensor and the motion sensor are part of different sub-systems, wherein
- the motion sensor system is designed to detect the relative movement or relative positions of the first body part and the second body part over a movement range, for example by generating data from which the movement trajectory or path of the relative movement of the first body part and the second body part can be obtained or is obtained, and
- the calibration sensor system is designed to determine the relative position of the first body part relative to the second body part if - for example only if and/or whenever - the first body part and the second body part are arranged relative to each other in a calibration range, wherein the calibration range optionally comprises the occlusion position between the upper and lower jaw and/or a region adjacent to the occlusion position,
wherein the first sub-system has a first sub-system carrier, which is designed for attachment to the first body part and/or
wherein the second sub-system has a second sub-system carrier, which is designed for attachment to the second body part and
wherein the first sub-system carrier and/or the second sub-system carrier is/are designed to function as carriers for at least one component of the calibration sensor system and the motion sensor system.

2. System according to Claim 1, wherein
a) the calibration sensor system and/or the motion sensor system is/are designed to determine the relative position in 6 degrees of freedom (six coordinates: three in rotation, three in translation), and/or wherein
b) the system is designed to compare the current relative position obtained by means of the calibration sensor system with a relative position obtained by means of the motion sensor system, preferably obtained simultaneously, in order to carry out a calibration adjustment, wherein preferably
b1), in particular if or only if the calibration adjustment shows that the compared relative positions are different, the system is designed to transmit the current relative position determined by means of the calibration sensor system to the motion sensor system so that the motion sensor system can use the current relative position as a starting point for the further movement detection;
and/or wherein
b2) the system is designed - in particular if or only if the calibration adjustment shows that the compared relative positions are different - to modify the trajectory determined by means of the motion sensor system based on the current relative position prior to the calibration adjustment such that the trajectory passes through the current relative position, and/or wherein
b3) the system is designed so that the modification comprises smoothing of the trajectory, in particular limited to a region close to the current relative position and/or wherein
b4) the system is designed so that the modification adapts the trajectory to a natural movement, and/or wherein
c) the calibration sensor system and/or the motion sensor system is/are designed to determine the movement trajectory or path in 6 degrees of freedom (six coordinates: three in rotation, three in translation).

3. System according to at least any selected preceding claim or any selected plurality of preceding claims,
wherein the system is designed to use data obtained by means of the calibration sensor system to verify data obtained by means of the motion sensor system.

4. System according to at least any selected preceding claim or any selected plurality of preceding claims,
wherein the movement range comprises all relative positions that can be assumed by the first and second body parts relative to one another, in particular the relative positions in the normal course of movement, and/or
wherein the calibration range is a proper subset of the movement range and/or wherein the calibration range is smaller than or equal to one of the following percentages of the movement range: 30%, 25%, 20%, 15%.

5. System according to at least any selected preceding claim or any selected plurality of preceding claims,
wherein the calibration sensor system has a measurement accuracy in the calibration range that is greater than the measurement accuracy of the motion sensor system in the movement range and in the calibration range and/or
wherein the calibration sensor system has a resolution in the calibration range that is at least twice as high as the average resolution of the motion sensor system in the movement range.

6. System according to at least any selected preceding claim or any selected plurality of preceding claims,
wherein the calibration sensor is, for example, a magnetic field sensor, wherein the calibration sensor system optionally has at least one calibration sensor exciter, which is designed to generate a sensor signal in the calibration sensor, wherein the calibration sensor exciter is, for example, a magnet, in particular with an anisotropic magnetic field matched to the magnetic field sensor.

7. System according to at least any selected preceding claim or any selected plurality of preceding claims,
wherein the calibration sensor is part of the first sub-system or the second sub-system and wherein the calibration sensor exciter is part of the other sub-system than the calibration sensor.

8. System according to at least any selected preceding claim or any selected plurality of preceding claims,
wherein the calibration sensor system is free of sensor exciters.

9. System according to at least any selected preceding claim or any selected plurality of preceding claims, wherein
a) the motion sensor is, for example, an inertial sensor or an optical sensor, and/or
b) the motion sensor system has at least one motion sensor exciter, which is designed to generate a sensor signal in the motion sensor, wherein the motion sensor exciter is, for example, one of the following exciters: radiation source, in particular a radiation source matched to the optical sensor, and/or
c) the motion sensor is part of the first sub-system or the second sub-system, and/or
d) the motion sensor exciter is part of the other sub-system than the motion sensor.

10. System according to at least any selected preceding claim or any selected plurality of preceding claims,
wherein the motion sensor system is free of sensor exciters.

11. System according to at least any selected preceding claim or any selected plurality of preceding claims,
wherein the calibration sensor and the motion sensor are part of the same sub-system and preferably attached to a common sub-system carrier.

12. System according to at least any selected preceding claim or any selected plurality of preceding claims,
a) comprising a plurality of calibration sensors, of which a first calibration sensor is part of the first sub-system and a second calibration sensor is part of the second sub-system, and/or
b) comprising a plurality of motion sensors, of which a first motion sensor is part of the first sub-system and a second motion sensor is part of the second sub-system, and/or
c) wherein the motion sensor and/or the calibration sensor is an integral sensor, and/or
d) wherein the calibration sensor and/or the motion sensor is a non-contact or contactless sensor or wherein the calibration sensor and/or the motion sensor is a contact or touch sensor, and/or
e) wherein the system comprises a processor device, in particular a microprocessor, which is designed:
- to control the motion sensor system and/or the calibration sensor system, and/or
- to carry out the comparison of the relative positions, and/or
f) wherein the system has an electrical power source, preferably a battery, and/or
g) wherein the system has a storage device, in particular a data storage device, wherein
g1) the storage device is optionally designed for the intermediate storage of data of the motion sensor system, and/or
g2) the system is designed such that it deletes data stored in the storage device after the calibration adjustment, preferably after correction or adjustment of the data of the motion sensor system, for example the trajectory, to the current relative position and/or before the next calibration adjustment, and/or wherein
h) the system comprises a spatial position sensor system, which is designed to determine the absolute position of the first body part or the second body part in space, in particular in 6 degrees of freedom, wherein optionally
h1) the spatial position sensor system is a sensor system without a sensor exciter, and/or
h2) the spatial position sensor system has a spatial position sensor, and/or wherein
i) the system comprises an orientation sensor system, which is designed to determine the orientation of the first body part or the second body part in space, wherein the orientation sensor system optionally comprises an orientation sensor, for example a gravity sensor.

13. System according to at least any selected preceding claim or any selected plurality of preceding claims,
wherein any component, any selected plurality of components or all of the following components of the system are designed to be arranged intraorally when the system is in operation:
- motion sensor(s)
- calibration sensor(s)
- spatial position sensor,
- gravity sensor,
- processor device,
- electrical power source,
- storage device, and
wherein optionally any component, any selected plurality of components is designed to be arranged extraorally when the system is in operation:
- motion sensor(s)
- calibration sensor(s)
- spatial position sensor,
- gravity sensor,
- processor device,
- electrical power source,
- storage device.

## Revendications

1. Système (100) de détection de mouvements du mouvement relatif de parties du corps, avec au moins deux sous-systèmes (20, 30), à savoir un premier sous-système et un deuxième sous-système, le premier sous-système étant destiné à être fixé à une première partie du corps, à savoir la mâchoire supérieure ou à une zone du corps solidaire de la mâchoire supérieure de manière rigide, comme le crâne, et le deuxième sous-système étant destiné à être fixé à une deuxième partie du corps, à savoir la mâchoire inférieure, dans lequel la première partie du corps et la deuxième partie du corps sont mobiles l'une par rapport à l'autre, et dans lequel le système comporte un système de capteurs de mouvements (621, 681, 631, 691) et un système de capteurs d'étalonnage (626, 636), le système de capteurs de mouvements et le système de capteurs d'étalonnage étant des systèmes de capteurs différents,
dans lequel le système de capteurs de mouvements comporte au moins un capteur de mouvements et dans lequel le système de capteurs d'étalonnage comporte au moins un capteur d'étalonnage,
dans lequel le capteur d'étalonnage et le capteur de mouvements font partie de sous-systèmes différents
- le système de capteur de mouvements étant conçu pour détecter le mouvement relatif ou les positions relatives de la première partie du corps et de la deuxième partie du corps sur une zone de mouvements, par exemple en générant des données à partir desquelles la trajectoire de mouvement ou la trajectoire du mouvements relatif de la première partie du corps et de la deuxième partie du corps peut être obtenue ou est obtenue, et
- le système de capteur d'étalonnage étant conçu pour déterminer la position relative de la première partie du corps relativement à la deuxième partie du corps, si - par exemple seulement si et/ou toujours si - la première partie du corps et la deuxième partie du corps sont disposées l'une par rapport à l'autre dans une zone d'étalonnage, optionnellement la zone d'étalonnage comprenant la position d'occlusion entre la mâchoire supérieure et la mâchoire inférieure et/ou comprenant une zone adjacente à la position d'occlusion, le premier sous-système comportant un premier support de sous-système conçu pour être fixé à la première partie du corps et/ou
le deuxième sous-système comportant un deuxième support de sous-système conçu pour être fixé à la deuxième partie du corps et
le premier support de sous-système et/ou le deuxième support de sous-système étant conçus pour faire office de support pour au moins un composant du système de capteur d'étalonnage et du système de capteurs de mouvements.

2. Système selon la revendication 1, dans lequel
a) le système de capteur de d'étalonnage et/ou le système de capteurs de mouvements sont conçu(s) pour déterminer la position relative en 6-Degrees-of-Freedom (six coordonnées : trois en rotation, trois en translation), et/ou dans lequel
b) le système est conçu pour comparer la position relative actuelle obtenue au moyen du système de capteurs d'étalonnage, à une position relative obtenue, de préférence simultanément, au moyen du système de capteurs de mouvements afin d'effectuer un ajustement d'étalonnage, dans lequel de préférence
b1), en particulier si ou seulement si l'ajustement d'étalonnage révèle que les positions relatives comparées sont différentes, le système est conçu pour transmettre la position relative actuelle déterminée au moyen du système de capteurs d'étalonnage, au système de capteurs de mouvement, afin que le système de capteurs de mouvements puisse utiliser la position relative actuelle comme point de départ pour la poursuite de la détection de mouvements ;
et/ou dans lequel
b2) le système est conçu - en particulier si ou seulement si l'ajustement d'étalonnage révèle que les positions relatives comparées sont différentes -, pour modifier, sur la base de la position relative actuelle, la trajectoire déterminée au moyen du système de capteurs de mouvements avant l'ajustement d'étalonnage, de sorte que la trajectoire passe par la position relative actuelle, et/ou dans lequel
b3) le système est conçu pour que la modification comprenne un lissage de la trajectoire, en particulier limité à une zone proche de la position relative actuelle, et/ou dans lequel
b4) le système est conçu pour que la modification adapte la trajectoire à un mouvement naturel, et/ou dans lequel
c) le système de capteurs d'étalonnage et/ou le système de capteurs de mouvements est/sont conçu(s) pour déterminer la trajectoire de mouvements ou la trajectoire en 6-Degrees-of-Freedom (six coordonnées : trois en rotation, trois en translation).

3. Système selon au moins une revendication précédente choisie arbitrairement ou une pluralité de revendications précédentes choisie arbitrairement, dans lequel le système est conçu pour utiliser des données obtenues au moyen du système de capteur d'étalonnage pour vérifier des données obtenues au moyen du système de capteur de mouvement.

4. Système selon au moins une revendication précédente choisie arbitrairement ou une pluralité de revendications précédentes choisie arbitrairement,
dans lequel la zone de mouvements comprend toutes les positions relatives pouvant être prises les unes par rapport aux autres par la première et la deuxième partie du corps, en particulier les positions relatives dans le déroulement normal du mouvement, et/ou
dans lequel la zone d'étalonnage est un sous-ensemble réel de la zone de mouvements et/ou dans lequel la zone d'étalonnage est inférieure ou égale à l'un des pourcentages suivants de la zone de mouvements : 30%, 25%, 20%, 15%.

5. Système selon au moins une revendication précédente choisie arbitrairement ou une pluralité de revendications précédentes choisie arbitrairement,
dans lequel le système de capteur d'étalonnage a une précision de mesure dans la zone d'étalonnage qui est supérieure à la précision de mesure du système de capteur de mouvement dans la zone de mouvement et dans la zone d'étalonnage et/ou
dans lequel le système de capteur d'étalonnage a une résolution dans la zone d'étalonnage qui est au moins 2 fois plus élevée que la résolution moyenne du système de capteur de mouvement dans la zone de mouvement.

6. Système selon au moins une revendication précédente choisie arbitrairement ou une pluralité de revendications précédentes choisie arbitrairement,
dans lequel le capteur d'étalonnage est par exemple un capteur de champ magnétique, dans lequel le système de capteur d'étalonnage comporte en option au moins un excitateur du capteur d'étalonnage qui est conçu pour générer un signal de capteur dans le capteur d'étalonnage, l'excitateur de capteur d'étalonnage étant par exemple un aimant, en particulier avec un champ magnétique anisotrope adapté au capteur de champ magnétique.

7. Système selon au moins une revendication précédente choisie arbitrairement ou une pluralité de revendications précédentes choisie arbitrairement,
dans lequel le capteur d'étalonnage fait partie du premier sous-système ou du deuxième sous-système et dans lequel l'excitateur de capteur d'étalonnage fait partie du sous-système autre que le capteur d'étalonnage.

8. Système selon au moins une revendication précédente choisie arbitrairement ou une pluralité de revendications précédentes choisie arbitrairement,
dans lequel le système de capteur d'étalonnage est dépourvu d'excitateur de capteur.

9. Système selon au moins une revendication précédente choisie arbitrairement ou une pluralité de revendications précédentes choisie arbitrairement, dans lequel
a) dans lequel le capteur de mouvements est par exemple un capteur inertiel ou un capteur optique, et/ou
b) le système de capteurs de mouvements comporte au moins un excitateur de capteur de mouvements conçu pour générer un signal de capteur dans le capteur de mouvements, l'excitateur de capteur de mouvements étant par exemple l'un des excitateurs suivants : source de rayonnement, en particulier une source de rayonnement adaptée au capteur optique, et/ou
c) le capteur de mouvements fait partie du premier sous-système ou du deuxième sous-système, et/ou
d) l'excitateur de capteur de mouvements fait partie du sous-système autre que le capteur de mouvements.

10. Système selon au moins une revendication précédente choisie arbitrairement ou une pluralité de revendications précédentes choisie arbitrairement,
dans lequel le système de capteur de mouvements est dépourvu d'excitateur de capteur.

11. Système selon au moins une revendication précédente choisie arbitrairement ou une pluralité de revendications précédentes choisie arbitrairement,
le capteur d'étalonnage et le capteur de mouvements faisant partie du même sous-système et étant de préférence fixés sur un support de sous-système commun.

12. Système selon au moins une revendication précédente choisie arbitrairement ou une pluralité de revendications précédentes choisie arbitrairement,
a) comprenant une pluralité de capteurs d'étalonnage, dont un premier capteur d'étalonnage fait partie du premier sous-système et un deuxième capteur d'étalonnage fait partie du deuxième sous-système, et/ou
b) comprenant une pluralité de capteurs de mouvements, dont un premier capteur de mouvements fait partie du premier sous-système et un deuxième capteur de mouvements fait partie du deuxième sous-système, et/ou
c) le capteur de mouvements et/ou le capteur d'étalonnage est un capteur intégral, et/ou
d) le capteur d'étalonnage et/ou le capteur de mouvements étant un capteur sans contact ou sans effleurement, ou le capteur d'étalonnage et/ou le capteur de mouvements étant un capteur à contact ou à effleurement, et/ou
e) le système comprenant un dispositif du processeur, en particulier un microprocesseur, conçu à cet effet pour :
- commander le système de capteurs de mouvements et/ou le système de capteurs d'étalonnage, et/ou
- effectuer la comparaison des positions relatives, et/ou
f) le système comportant une source d'énergie électrique, de préférence une batterie, et/ou
g) le système comportant un dispositif d'enregistrement, en particulier une mémoire de données, dans lequel
g1) le dispositif d'enregistrement est conçu, en option, pour stocker temporairement des données du système de capteurs de mouvements, et/ou
g2) le système est conçu pour effacer les données stockées dans le dispositif d'enregistrement après l'ajustement d'étalonnage, de préférence une fois la correction réussie ou après l'ajustement des données du système de capteurs de mouvements, par exemple la trajectoire, à la position relative actuelle et/ou avant l'ajustement d'étalonnage suivant, et/ou dans lequel
h) le système comprend un système de capteur de position spatiale conçu pour déterminer la position absolue de la première partie du corps ou de la deuxième partie du corps dans l'espace, en particulier en 6 degrés de liberté, dans lequel, en option
h1) le système de capteur de position spatiale est un système de capteurs dépourvu d'excitateur de capteur, et/ou
h2) le système de capteur de position spatiale comporte un capteur de position spatiale, et/ou dans lequel
i) le système comprend un système de capteur d'orientation conçu pour déterminer l'orientation de la première partie du corps ou de la deuxième partie du corps dans l'espace, le système de capteurs d'orientation comprenant optionnellement un capteur d'orientation, par exemple un capteur de gravité.

13. Système selon au moins une revendication précédente choisie arbitrairement ou une pluralité de revendications précédentes choisie arbitrairement,
dans lequel un composant quelconque, une pluralité de composants choisis arbitrairement ou tous les composants suivants du système sont conçus pour être placés intra-oralement lorsque le système est en fonctionnement :
- capteur(s) de mouvements,
- capteur(s) d'étalonnage,
- capteur de position spatiale,
- capteur de gravité,
- dispositif du processeur,
- source d'énergie électrique,
- dispositif d'enregistrement et
dans lequel, en option, un composant quelconque, une pluralité de composants choisis arbitrairement, est conçu pour être placé extra-oralement lorsque le système est en fonctionnement :
- capteur(s) de mouvements,
- capteur(s) d'étalonnage,
- capteur de position spatiale,
- capteur de gravité,
- dispositif du processeur,
- source d'énergie électrique,
- dispositif d'enregistrement.
